# EUROPEAN PATENT APPLICATION

(11) **EP 4 527 872 A1**
(43) Date of publication of application: **26.03.2025**
(21) Application number: 23814721.9
(22) Date of filing: 21.03.2023
(51) Int. Cl.: C08G 63/685, C08G 63/688, C08G 63/68, C08G 63/91, C08G 65/328, C08G 63/83

(54) **CATALYST FOR POLYESTER DEPOLYMERIZATION OR CYCLIC ESTER SYNTHESIS, PREPARATION METHOD THEREFOR AND USE THEREOF**

(30) Priority: 02.06.2022 CN 202210623079
(71) Applicant: Sichuan University, Chengdu, Sichuan 610065 (CN)
(72) Inventor: WANG, Yuzhong, Chengdu, Sichuan 610065 (CN); TIAN, Guoqiang, Chengdu, Sichuan 610065 (CN); CHEN, Sichong, Chengdu, Sichuan 610065 (CN); LUO, Zixuan, Chengdu, Sichuan 610065 (CN); LI, Maoqin, Chengdu, Sichuan 610065 (CN); LEI, Jin, Chengdu, Sichuan 610065 (CN)
(74) Representative: Kraus & Lederer PartGmbB
(86) International application number: PCT/CN2023/082814
(87) International publication number: WO 2023/231524

(57) **Abstract**

Disclosed herein are a catalyst for polyester depolymerization or cyclic ester synthesis, a preparation method therefor and a use thereof. The catalyst is a long-chain catalyst having a main chain carbon atom number greater than or equal to 8, and containing a terminal ion group and a terminal hydroxyl structure; the catalyst may be used to catalyze polyester hydrolysis, alcoholysis or cyclic depolymerization, so as to recover corresponding monomers or monomers and oligomers, or used to catalyze hydroxy acid or hydroxy acid esters to synthesize cyclic ester monomers or cyclic ester monomers and cyclic oligomers by means of polycondensation and cyclization reactions. The catalyst may reduce the viscosity of a reaction system, and may greatly improve the utilization rate of a catalytic active center, reduce the consumption of the catalyst and improve overall catalytic efficiency, achieving high-efficiency, high-yield and selective depolymerization of polyester homopolymers, copolymers, blends or compounds; operation is simple, and convenient for industrial production.

## Description

This application claims the priority of Chinese patent application No. 202210623079.8 filed on June 2, 2022, the content of which is incorporated herein by reference in its entirety as part of the present application.

### TECHNICAL FIELD

The present disclosure belongs to the technical field of polyester catalysts and preparation and use thereof. The present disclosure specifically relates to a catalyst for polyester depolymerization or cyclic ester synthesis, and a preparation method and use thereof.

### BACKGROUND

Synthetic polymer materials are widely used in production and life due to their advantages such as light weight, low price and good processability. With the large-scale production and use of polymer materials, the amount of waste is also increasing continuously, which not only consumes a lot of resources but also causes serious environmental pollution. Therefore, the development of recycling technology for polymer materials has become increasingly important and urgent.

Existing recycling methods include physical recycling for downgrading and reuse (CN201710740467.3), chemical recycling as high value-added products (CN201410233916.1), depolymerization and recovery as monomers (CN201510511713.9), etc. Among them, the method of depolymerization and recovery as monomers is that the recovered products, i.e., monomers and oligomers, can be used again to synthesize polymers with the same properties as those of the original products, thus forming a closed-loop cycle process, which has unique advantages in reducing resource consumption and environmental pollution (Nature Chemistry, 2016, 8: 42-49)(Science, 2018, 360: 398-403)(Nature, 2021, 590: 423-427)(Science, 2021, 373: 783-789).

However, the current depolymerization and recovery methods of polyesters and the cyclization reactions of cyclic ester synthesis still have the following problems when entering the middle and late stages of the reaction: (1) The high viscosity of polyesters will lead to serious mass transfer problems during the reaction, posing a great challenge to the reaction equipment and design of processes. The existing catalysts and their catalytic methods cannot achieve viscosity reduction effects while catalyzing the reaction, resulting in not only a slow reaction rate but also a high content of impurity in the product; (2) The catalysts currently used often have poor compatibility with polyesters and thus are difficult to fully exert their catalytic effect, resulting in high catalyst consumption, low depolymerization efficiency, and high depolymerization cost; (3) When there are chiral sites in the reactants, the existing catalytic methods often require the use of extreme conditions such as strong acid, strong base, and high temperature; therefore, stereoisomerization reactions will occur during the reaction, resulting in racemization of the product and low utilization value.

### SUMMARY

One aspect of the present disclosure is to provide a catalyst for polyester depolymerization or cyclic ester synthesis to address the problems existing in the current depolymerization and recovery and cyclic ester synthesis.

Another aspect of the present disclosure is to provide a method of preparing the above catalyst for polyester depolymerization or cyclic ester synthesis.

Another aspect of the present disclosure is to provide use of the above catalyst for polyester depolymerization or cyclic ester synthesis.

The present disclosure provides a catalyst for polyester depolymerization or cyclic ester synthesis, which is a long-chain catalyst containing both a terminal ion group and a terminal hydroxyl structure, wherein the terminal ion group is either a cation connected to the long-chain structure or an anion connected to the long-chain structure, and the structural formulas are respectively as follows: 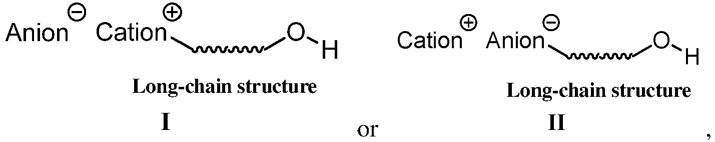 the number of carbon atoms in the main chain of the long-chain structure is 8 or more; the long-chain structure of the structural formula I is any one of a long alkyl chain, an aliphatic polyester chain and a polyether-ester chain; the long-chain structure of the structural formula II is any one of a long alkyl chain, an aliphatic polyester chain, a polyether-ester chain and a polyether chain.

For example, the long alkyl chain of the long-chain structure in the catalyst is an n-octane chain, an n-pentadecane chain or an α-hexyldodecane chain; the aliphatic polyester chain is poly(ethylene succinate), polybutylene succinate, poly(hexylene succinate), poly(ethylene adipate), polybutylene adipate, poly(hexylene adipate), polylactic acid, polyglycolide, polycaprolactone or polyvalerolactone; the polyether-ester chain is polydioxanone, poly(3,4-dihydro-2H-benzo[1,4]dioxepine-2-one) or poly(4-phenyl-3,4-dihydro-2H-benzo[1,4]dioxepine-2-one); and the polyether chain is polyethylene glycol or polytetrahydrofuran.

For example, in the catalyst, the cation in the above structural formula I of the catalyst is an imidazolium ion or a thiazolium ion, and the anion is a halide ion or a halogenated metal chloride; the anion in the structural formula II is an organic carboxylate ion or an organic sulfonate ion, and the cation is a metal ion or a quaternary ammonium ion.

For example, in the catalyst, the imidazolium ion in the above structural formula I of the catalyst is a 1-long-chain substituted-3-methylimidazolium ion or a 1-long-chain substituted-3-butylimidazolium ion, the thiazolium ion is a 3-long-chain substituted-4-methylthiazolium ion, a 3-long-chain substituted benzothiazolium ion or a 3-long-chain substituted thiazolium ion; the halide ion in the structural formula I is a chloride ion or a bromide ion; the halogenated metal chloride in the structural formula I is chlorinated stannous chloride, chlorinated aluminum chloride, chlorinated zinc chloride, brominated ferric chloride or chlorinated ferric chloride; the organic carboxylate ion in the structural formula II is an aliphatic carboxylate ion; the organic sulfonate ion in the structural formula II is an aliphatic sulfonate ion; the metal ion in the structural formula II is a sodium ion, a potassium ion, a magnesium ion, a calcium ion, a zinc ion, a tin ion, an iron ion or an aluminum ion; the quaternary ammonium ion in the structural formula II is a tetramethylammonium ion or a tetraethylammonium ion.

The present disclosure also provides a method of preparing a catalyst for polyester depolymerization or cyclic ester synthesis, wherein the process steps and conditions of the preparation method one are as follows:
(1) subjecting a hydroxyl-containing alkyl halide to a quaternization reaction with 0.8-1.2 times the molar amount of a compound containing an imidazole or thiazole structure to give a hydroxyl-containing imidazolium halide, a hydroxyl-containing thiazolium halide, a hydroxyl-containing imidazolium halide catalyst with a long alkyl chain in the structural formula I, or a hydroxyl-containing thiazolium halide catalyst with a long alkyl chain in the structural formula I;
(2) subjecting the hydroxyl-containing imidazolium halide, hydroxyl-containing thiazolium halide, hydroxyl-containing imidazolium halide catalyst with a long alkyl chain in the structural formula I or hydroxyl-containing thiazolium halide catalyst with a long alkyl chain in the structural formula I obtained in step (1) to a Lewis acid-base neutralization reaction with 1-3 times the molar amount of a Lewis acidic metal ion chloride, to convert the anion of the above salt from a halide ion to a halogenated metal chloride, thereby giving another type of hydroxyl-containing imidazolium salt, another type of hydroxyl-containing thiazolium salt, another type of hydroxyl-containing imidazolium salt catalyst with a long alkyl chain in the structural formula I or another type of hydroxyl-containing thiazolium salt catalyst with a long alkyl chain in the structural formula I;
(3) subjecting the other type of hydroxyl-containing imidazolium salt, the other type of hydroxyl-containing thiazolium salt, the other type of hydroxyl-containing imidazolium salt catalyst with a long alkyl chain in the structural formula I or the other type of hydroxyl-containing thiazolium salt catalyst with a long alkyl chain in the structural formula I obtained in step (2) to a ring-opening polymerization reaction with 1-100 times the molar amount of a cyclic ester or a cyclic ether-ester to give an imidazolium salt catalyst with a long-chain structure of polyester or polyether-ester in the structural formula I or a thiazolium salt catalyst with a long-chain structure of polyester or polyether-ester in the structural formula I; or mixing the other type of hydroxyl-containing imidazolium salt, the other type of hydroxyl-containing thiazolium salt, the other type of hydroxyl-containing imidazolium salt catalyst with a long alkyl chain in the structural formula I or the other type of hydroxyl-containing thiazolium salt catalyst with a long alkyl chain in the structural formula I obtained in step (2) with 1-100 times the molar amount of a hydroxy acid or with 1-100 times the molar amount of a dibasic acid and 1-100 times the molar amount of a diol to give an imidazolium salt or thiazolium salt catalyst with a long-chain structure of aliphatic polyester in the structural formula I through an esterification polycondensation reaction,

the process steps and conditions of the preparation method two are as follows:
   (1) mixing a hydroxyl-containing organic carboxylic acid or a hydroxyl-containing organic sulfonic acid with an alkali metal hydroxide, an alkaline earth metal hydroxide or a quaternary ammonium base at a molar ratio of carboxylate or sulfonate to hydroxide of 0.8-1.2, and preparing a hydroxyl-containing organic carboxylate, organic sulfonate, a hydroxyl-containing organic carboxylate catalyst with a long alkyl chain in the structural formula II or a hydroxyl-containing organic sulfonate catalyst with a long alkyl chain in the structural formula II whose cation is an alkali metal ion, an alkaline earth metal ion or a quaternary ammonium ion through a Bronsted-Lowry acid-base neutralization reaction; or mixing a cyclic ester with an alkali metal hydroxide, an alkaline earth metal hydroxide or a quaternary ammonium base at a molar ratio of cyclic ester to hydroxide of 0.8-1.2, and preparing a hydroxyl-containing organic carboxylate or a hydroxyl-containing organic sulfonate catalyst with a long alkyl chain in the structural formula II whose cation is an alkali metal ion, an alkaline earth metal ion or a quaternary ammonium ion through an ester bond alkaline hydrolysis reaction;
   (2) mixing the organic carboxylate, organic sulfonate, hydroxyl-containing organic carboxylate catalyst with a long alkyl chain in the structural formula II or hydroxyl-containing organic sulfonate catalyst with a long alkyl chain in the structural formula II whose cation is an alkali metal ion obtained in step (1) with a Lewis acidic metal ion chloride at a molar ratio of carboxylate or sulfonate to chloride ion of 0.8-2.2, and preparing another type of hydroxyl-containing organic carboxylate, organic sulfonate, hydroxyl-containing organic carboxylate catalyst with a long alkyl chain in the structural formula II or hydroxyl-containing organic sulfonate catalyst with a long alkyl chain in the structural formula II whose cation is a Lewis acidic metal ion through a metathesis reaction;
   (3) mixing the hydroxyl-containing organic carboxylate, organic sulfonate, hydroxyl-containing organic carboxylate catalyst with a long alkyl chain in the structural formula II, or hydroxyl-containing organic sulfonate catalyst with a long alkyl chain in the structural formula II whose cation is an alkali metal ion, an alkaline earth metal ion, a quaternary ammonium ion or a Lewis acidic metal ion obtained in step (2) with 1-100 times the molar amount of a cyclic ester or a cyclic ether-ester to give a catalyst with a long-chain structure of polyester or polyether-ester in the structural formula II through a ring-opening polymerization reaction, or with 1-100 times the molar amount of a hydroxy acid or 1-100 times the molar amount of a dibasic acid and 1-100 times the molar amount of a diol to give a catalyst with a long-chain structure of polyester in the structural formula II through an esterification polycondensation reaction,
the process steps and conditions of the preparation method three are as follows:
   (1) mixing an aliphatic polyester or polyether-ester with an alkali metal hydroxide, an alkaline earth metal hydroxide or a quaternary ammonium base at a molar ratio of ester bond to hydroxide of 1-100, and then performing an ester bond alkaline hydrolysis reaction at a temperature higher than the melting point of the corresponding polymer to give an aliphatic polyester or polyether-ester catalyst containing a terminal carboxylate and a terminal hydroxyl whose cation is an alkali metal ion, an alkaline earth metal ion or a quaternary ammonium ion in the structural structure II; or mixing a polyether containing a terminal carboxylic acid and a terminal hydroxyl with an alkali metal hydroxide, an alkaline earth metal hydroxide or a quaternary ammonium base at a molar ratio of terminal carboxylate to hydroxide of 0.8-1.2, and then performing a Bronsted-Lowry acid-base neutralization reaction at a temperature higher than the melting point of the corresponding polymer to give an aliphatic polyester, polyether-ester or polyether catalyst containing a terminal carboxylate and a terminal hydroxyl whose cation is an alkali metal ion, an alkaline earth metal ion or a quaternary ammonium ion in the structural formula II;
   (2) mixing the aliphatic polyester, polyether-ester or polyether catalyst containing a terminal carboxylate and a terminal hydroxyl whose cation is an alkali metal ion in the structural formula II obtained in step (1) with a Lewis acidic metal ion chloride at a molar ratio of carboxylate to chloride ion of 1-2, and then performing a metathesis reaction at a temperature higher than the melting point of the corresponding polymer to give an aliphatic polyester, polyether-ester or polyether catalyst containing a terminal carboxylate and a terminal hydroxyl whose cation is a Lewis acidic metal ion in the structural formula II.

For example, the hydroxyl-containing alkyl halide used in the quaternization reaction described in the method one is 8-chloro-1-octanol, 3-chloro-1-propanol or 2-bromo-1-ethanol, and the imidazole or thiazole structure-containing compound used is 1-methylimidazole, 1-butylimidazole, 4-methylthiazole, thiazole or benzothiazole, the reaction temperature is 60-120 °C, and the reaction time is 12-48 h;
the MClₙ used in the Lewis acid-base neutralization reaction described in the method one is zinc chloride, stannous chloride, aluminum chloride or ferric chloride, the reaction temperature is 25-120 °C, and the reaction time is 0.5-12 h;
the cyclic ester or cyclic ether-ester used in the ring-opening polymerization reaction described in the method one or method two is lactide, glycolide, ε-caprolactone, δ-valerolactone, p-dioxanone, 3,4-dihydro-2H-benzo[1,4]dioxepine-2-one or 4-phenyl-3,4-dihydro-2H-benzo[1,4]dioxepine-2-one, the reaction temperature is 60-220 °C, and the reaction time is 1-48 h;
the dibasic acid used in the esterification polycondensation reaction described in the method one or method two is 1,4-butanedioic acid or 1,6-hexanedioic acid, the diol used is 1,2-ethanediol, 1,4-butanediol or 1,6-hexanediol, the reaction temperature is 80-220 °C, the reaction time is 8-24 h, and the reaction pressure is 1-1*10⁵ Pa;
the hydroxyl-containing organic carboxylic acid used in the Bronsted-Lowry acid-base neutralization reaction described in the method two is 12-hydroxyoctadecanoic acid, 6-hydroxyhexanoic acid, 18-hydroxyoctadecanoic acid or 8-hydroxyoctanoic acid, the hydroxyl-containing organic sulfonic acid used is 4-hydroxy-1-butanesulfonic acid, 3-hydroxy-1-propanesulfonic acid or 2-hydroxyethanesulfonic acid, the alkali metal hydroxide used is sodium hydroxide or potassium hydroxide, the alkaline earth metal hydroxide used is magnesium hydroxide or calcium hydroxide, the quaternary ammonium base used is tetramethylammonium hydroxide or tetraethylammonium hydroxide, the reaction temperature is 25-180 °C, and the reaction time is 0.5-12 h;
the cyclic ester used in the ester bond alkaline hydrolysis reaction described in the method two is cyclopentadecanolide or ε-CL, the alkali metal hydroxide used is sodium hydroxide or potassium hydroxide, the alkaline earth metal hydroxide used is magnesium hydroxide or calcium hydroxide, the quaternary ammonium base used is tetramethylammonium hydroxide or tetraethylammonium hydroxide, the reaction temperature is 25-180 °C, and the reaction time is 0.5-12 h;
the MClₙ used in the metathesis reaction described in the method two or method three is ZnCl₂, SnCl₂, AlCl₃ or FeCl₃, the reaction temperature is 25-220 °C, and the reaction time is 0.5-12 h;
the aliphatic polyester used in the ester bond alkaline hydrolysis reaction described in the method three is PES, PBS, PHS, PEA, PBA, PHA, PLA, PGA, PCL or PVL, the polyether-ester used is PPDO, PBDXO or PDBXOP, the reaction temperature is 60-220 °C, and the reaction time is 1-48 h;
the polyether used in the Bronsted-Lowry acid-base neutralization reaction described in the method three is PEG or PTHF, the reaction temperature is 25-120 °C, and the reaction time is 0.5-12 h.

The present disclosure also provides use of the catalyst for polyester depolymerization or cyclic ester synthesis, wherein the use is for catalyzing the hydrolysis, alcoholysis or cyclodepolymerization of a polyester homopolymer, copolymer, blend or complex to recover the corresponding monomers or monomers and oligomers, or for catalyzing the synthesis of a cyclic ester monomer or a cyclic ester monomer and cyclic oligomer from hydroxy acids or hydroxy esters through a polycondensation and cyclization reaction.

For example, the specific method for the use for catalyzing the hydrolysis, alcoholysis or cyclodepolymerization of a polyester homopolymer, copolymer, blend or complex to recover the corresponding monomers or monomers and oligomers is as follows: mixing the polyester, water or alcohol, and catalyst according to a proportion, heating the mixture to 40-200 °C at 0.1-2 MPa for a hydrolysis or alcoholysis reaction for 1-12 h, and then recovering a solution of the corresponding monomers or monomers and oligomers in water or alcohol; or heating the mixture to 80-400 °C at 1-1*10⁴ Pa for cyclodepolymerization for 0.5-12 h, and simultaneously distilling or extracting the mixture to separate the generated cyclic ester monomers or cyclic ester monomers and cyclic oligomers from the reaction system; wherein the ratio of polyester to water or alcohol is 100:50-2000 wt%, and the molar ratio of the catalyst to the ester bond in the polyester is 0.01-10 mol% based on the terminal hydroxyl.

For example, the specific method for the use for catalyzing the synthesis of a cyclic ester monomer or cyclic ester monomer and cyclic oligomer from hydroxy acids or hydroxy esters through a polycondensation and cyclization reaction is as follows:
(1) first, mixing the catalyst with a hydroxy acid or hydroxy ester at a molar ratio of 0.01-10 mol% based on the terminal hydroxyl, and then heating the mixture to 80-220 °C at 100-1*10⁵ Pa for a polycondensation reaction for 4-12 h, and simultaneously distilling the mixture to remove the water or alcohol generated in the reaction system to give an oligomer of the hydroxy acid;
(2) heating the obtained oligomer of hydroxy acid to 80-400 °C at 1-1*10⁵ Pa for a cyclodepolymerization reaction for 0.5-4 h, and simultaneously distilling or extracting the resulting mixture to separate the generated cyclic ester monomers or cyclic ester monomers and cyclic oligomers from the reaction system.

For example, in the method of catalyzing the synthesis of a cyclic ester monomer or a cyclic ester monomer and a cyclic oligomer, the hydroxy acid is glycolic acid, lactic acid, 6-hydroxyhexanoic acid, 5-hydroxypentanoic acid or 12-hydroxyoctadecanoic acid, and the hydroxy ester is any one of methyl glycolate, ethyl glycolate, methyl lactate, ethyl lactate, methyl 6-hydroxyhexanoate, ethyl 6-hydroxyhexanoate, methyl 5-hydroxypentanoate, ethyl 5-hydroxypentanoate, methyl 12-hydroxyoctadecanoate and ethyl 12-hydroxyoctadecanoate.

### BRIEF DESCRIPTION OF DRAWINGS

In order to illustrate the technical solutions of the examples of the present disclosure more clearly, the drawings of the examples will be briefly introduced below. Obviously, the drawings in the following description only relate to some examples of the present disclosure, rather than limiting the present disclosure.

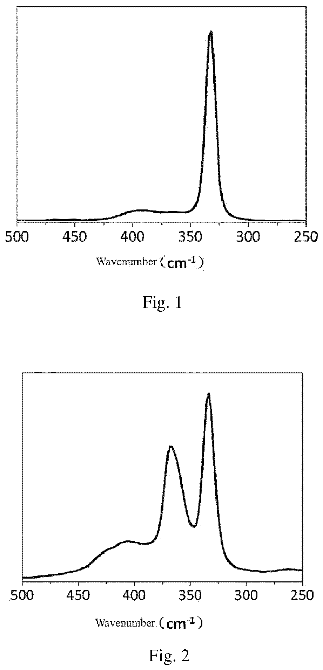

Fig. 1 is the laser Raman spectrum of the product of step (3) of example 1 of the present disclosure. The absorption peak at 330 cm⁻¹ in the figure is attributed to FeCl₃-Cl⁻. The appearance of this absorption peak indicates that the anion of the prepared catalyst is converted from Cl⁻ to FeCl₃-Cl⁻.
Fig. 2 is the laser Raman spectrum of the product of step (3) of example 11 of the present disclosure. The absorption peak at 330 cm⁻¹ in the figure is attributed to FeCl₃-Cl⁻, and the absorption peaks at 370 and 420 cm⁻¹ are attributed to 2FeCl₃-Cl⁻. The appearance of these two types of absorption peaks indicates that the anion of the prepared catalyst is converted from Cl to a mixture of FeCl₃-Cl⁻ and 2FeCl₃-Cl⁻. According to the ratio of FeCl₃ used, the mixture of FeCl₃-Cl⁻ and 2FeCl₃-Cl⁻ is recorded as 1.7FeCl₃-Cl⁻.

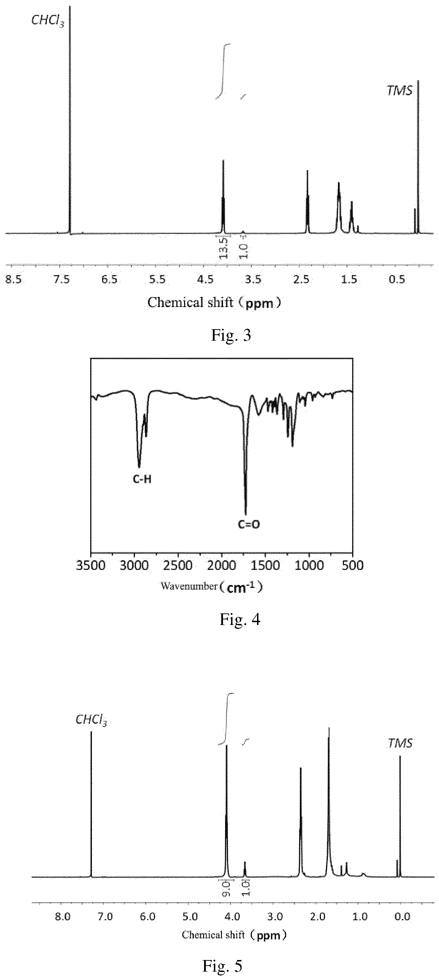

Fig. 3 is the ¹H NMR spectrum of the product of step (2) of example 69 of the present disclosure. On the one hand, it can be known from the analysis of the spectrum that the main component of the product is PCL, and on the other hand, the average degree of polymerization of the product can be calculated to be 14.5 according to the integral area ratio in the figure.
Fig. 4 is the fourier transform infrared spectrum of the product of step (2) of example 69 of the present disclosure. According to the analysis of the absorption peak of ester bond carbonyl and the absorption peak of carbon-hydrogen bond, etc., in the figure, it can be auxiliaryly explained that the main component of the product is an aliphatic polyester.
Fig. 5 is the ¹H NMR spectrum of the product of step (2) of example 70 of the present disclosure. On the one hand, it can be known from the analysis of the spectrum that the main component of the product is PVL, and on the other hand, the average degree of polymerization can be calculated to be 10 according to the integral area ratio.

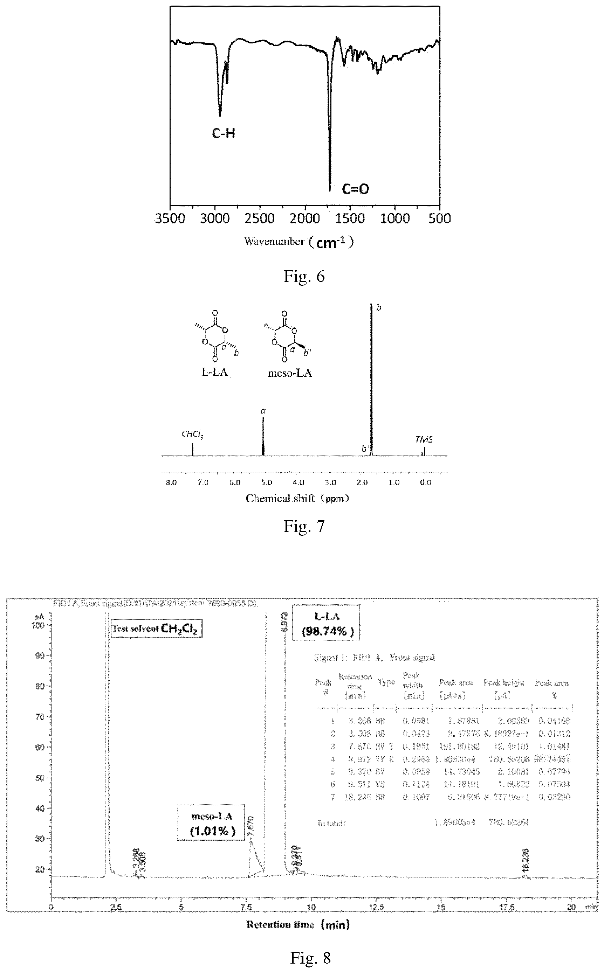

Fig. 6 is the fourier transform infrared spectrum of the product of step (2) of example 70 of the present disclosure. According to the analysis of the absorption peak of ester bond carbonyl and the absorption peak of carbon-hydrogen bond, etc., in the figure, it can be auxiliaryly explained that the main component of the product is an aliphatic polyester.
Fig. 7 is the ¹H NMR spectrum of the product of cyclodepolymerization in application example 22 of the present disclosure. According to the analysis of the spectrum, it can be seen that the product is mainly L-lactide (L-LA), and also contains a small amount of meso-lactide (meso-LA).
Fig. 8 is the gas chromatogram of the product of cyclodepolymerization in application example 22 of the present disclosure. According to the gas chromatography analysis, the proportions of L-LA and meso-LA in the recovered product are 98.74% and 1.01%, respectively.

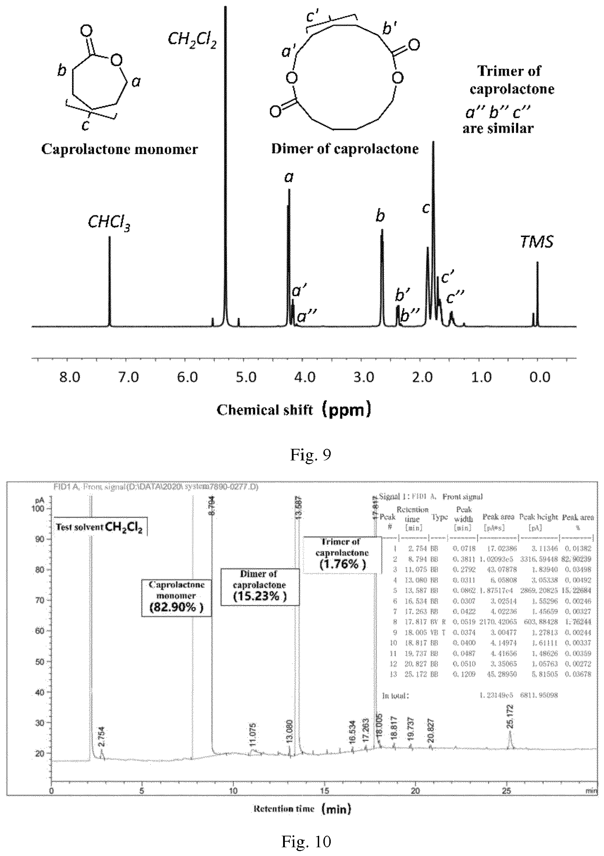

Fig. 9 is the ¹H NMR spectrum of the product of cyclodepolymerization in application example 24 of the present disclosure. According to the analysis of the spectrum, it can be seen that the product is a mixture of ε-CL monomers, cyclic dimers and cyclic trimers.
Fig. 10 is the gas chromatogram of the product of cyclodepolymerization in application example 24 of the present disclosure. According to the gas chromatography analysis, the proportions of ε-CL monomers, cyclic dimers and cyclic trimers in the recovered product are 82.90%, 15.23% and 1.76%, respectively.

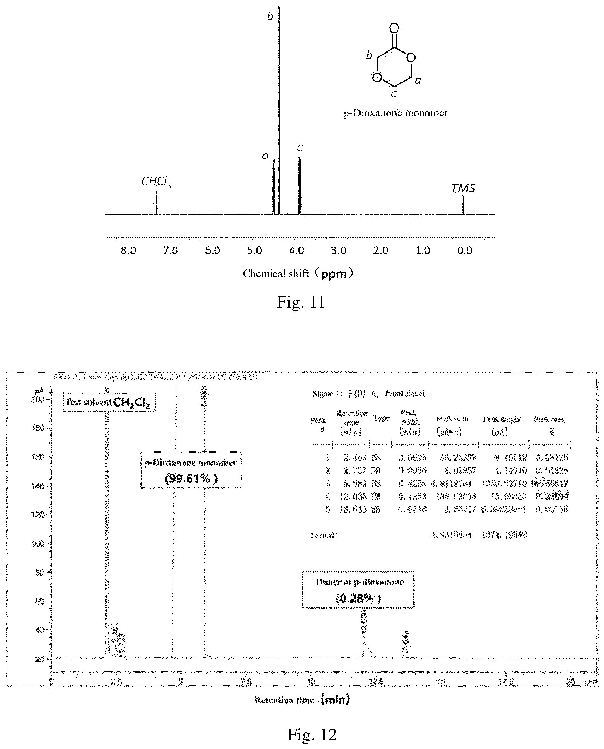

Fig. 11 is the ¹H NMR spectrum of the product of cyclodepolymerization in application example 25 of the present disclosure. According to the analysis of the spectrum, it can be known that the product is a high-purity PDO monomer.
Fig. 12 is the gas chromatogram of the product of cyclodepolymerization in application example 25 of the present disclosure. According to the gas chromatography analysis, the purity of the recovered PDO monomer is 99.61%, and 0.28% of cyclic dimers is also contained.

### DETAILED DESCRIPTION

In order to make the purpose, technical solutions and advantages of the examples of the present disclosure clearer, the technical solutions of the examples of the present disclosure will be clearly and completely described below in conjunction with the drawings of the examples of the present disclosure. Apparently, the described examples are some of the examples of the present disclosure, not all of them. Based on the described examples of the present disclosure, all other examples obtained by persons of ordinary skill in the art without creative efforts shall fall within the protection scope of the present disclosure.

The present disclosure may be embodied in other specific forms without departing from essential attributes of the present disclosure. It should be understood that any and all embodiments of the present disclosure can be combined with technical features in any other embodiment or multiple other embodiments to obtain additional embodiments under the premise of no conflict. The present disclosure includes further embodiments resulting from such combinations.

All publications and patents mentioned in this disclosure are hereby incorporated by reference into this disclosure in their entirety. To the extent that usage or terminology used in any publications and patents incorporated by reference conflicts with usage or terminology used in the present disclosure, the usage and terminology in the present disclosure shall prevail.

The section headings used herein are for the purpose of organizing the article only and should not be construed as limitations on the subject matter described.

Unless defined otherwise, all technical and scientific terms used herein have their ordinary meanings in the art to which the claimed subject matter belongs. In the event that more than one definition exists for a term, the definition herein shall prevail.

Except in the working examples or where otherwise indicated, all numbers stating quantitative properties such as dosages in the specification and claims are to be understood as modified in all instances by the term "about". It is also to be understood that any numerical range recited herein is intended to include all subranges within that range and any combination of various endpoints of such ranges or subranges. For example, the integers 1-10 include 1, 2, 3, 4, 5, 6, 7, 8, 9, and 10, and also include subranges 1-3, 1-4, 1-9, 2-4, 2-10, etc.

The words "comprising", "including" or "containing" and similar words used in the present disclosure mean that the elements appearing before the word cover the elements listed after the word and their equivalents, and do not exclude unrecited elements. The terms "comprising" or "including (containing)" used herein can be open, semi-closed and closed. In other words, the terms also include "consisting essentially of", or "consisting of".

The catalyst for polyester depolymerization or cyclic ester synthesis provided by the present disclosure is a long-chain catalyst containing both a terminal ion group and a terminal hydroxyl structure, wherein the terminal ion group is either a cation connected to the long-chain structure or an anion connected to the long-chain structure, and the structural formulas are respectively as follows: 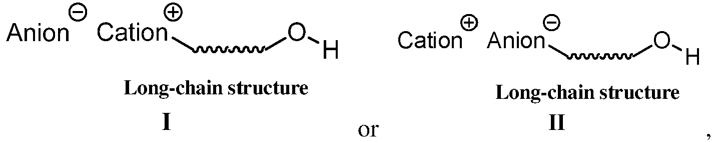 wherein, the number of carbon atoms in the main chain of the long-chain structure is 8 or more; the long-chain structure of the structural formula I is any one of a long alkyl chain, an aliphatic polyester chain and a polyether-ester chain; the long-chain structure of the structural formula II is any one of a long alkyl chain, an aliphatic polyester chain, a polyether-ester chain and a polyether chain.

The long alkyl chain of the long-chain structure in the above catalyst is, for example, an n-octane chain, an n-pentadecane chain or an α-hexyldodecane chain; the aliphatic polyester chain is, for example, poly(ethylene succinate) (PES), polybutylene succinate (PBS), poly(hexylene succinate) (PHS), poly(ethylene adipate) (PEA), polybutylene adipate (PBA), poly(hexylene adipate) (PHA), polylactic acid (PLA), polyglycolide (PGA), polycaprolactone (PCL) or polyvalerolactone (PVL); the polyether-ester chain is, for example, polydioxanone (PPDO), poly(3,4-dihydro-2H-benzo[1,4]dioxepine-2-one) (PBDXO) or poly(4-phenyl-3,4-dihydro-2H-benzo[1,4]dioxepine-2-one) (PDBXOP); and the polyether chain is, for example, polyethylene glycol (PEG) or polytetrahydrofuran (PTHF).

The cation in the structural formula I of the above catalyst is, for example, an imidazolium ion or a thiazolium ion, and the anion is, for example, a halide ion (X⁻) or a halogenated metal chloride (m·MClₙ-X⁻, wherein M represents a Lewis-acidic metal ion; n represents the number of chloride ions, which is equal to the valence state of the metal ion M; and m represents the molar ratio of the metal chloride to the halide ion, which is any value between 1 and 3); the anion in the structural formula II is, for example, an organic carboxylate ion or an organic sulfonate ion, and the cation is, for example, a metal ion or a quaternary ammonium ion.

The imidazolium ion in the above structural formula I of the catalyst is, for example, a 1-long-chain substituted-3-methylimidazolium ion or a 1-long-chain substituted-3-butylimidazolium ion, the thiazolium ion is, for example, a 3-long-chain substituted-4-methylthiazolium ion, a 3-long-chain substituted benzothiazolium ion or a 3-long-chain substituted thiazolium ion; the X⁻ in the structural formula I is, for example, a chloride ion (Cl⁻) or a bromide ion (Br⁻); the m·MClₙ-X⁻ in the structural formula I is, for example, chlorinated stannous chloride (m·SnCl₂-Cl⁻), chlorinated aluminum chloride (m·AlCl₃-Cl⁻), chlorinated zinc chloride (m·ZnCl₂-Cl⁻), brominated ferric chloride (m·FeCl₃-Br⁻) or chlorinated ferric chloride (m·FeCl₃-Cl⁻); the organic carboxylate ion in the structural formula II is, for example, an aliphatic carboxylate ion; the organic sulfonate ion in the structural formula II is, for example, an aliphatic sulfonate ion; the metal ion in the structural formula II is, for example, a sodium ion, a potassium ion, a magnesium ion, a calcium ion, a zinc ion, a tin ion, an iron ion or an aluminum ion; the quaternary ammonium ion in the structural formula II is, for example, a tetramethylammonium ion or a tetraethylammonium ion.

The number of carbon atoms in the main chain of the long-chain structure in the above catalyst is alternatively 8-1200.

Provided by the present disclosure is a method of preparing the above catalyst for polyester depolymerization or cyclic ester synthesis, wherein, the process steps and conditions of the preparation method one are as follows:
(1) subjecting a hydroxyl-containing alkyl halide to a quaternization reaction with 0.8-1.2 times the molar amount of a compound containing an imidazole or thiazole structure to give a hydroxyl-containing imidazolium halide (X^{- +}R'Im-R-OH, R' and R-OH are respectively the substituent groups on the two nitrogen atoms of the imidazole ring, wherein the number of carbon atoms in the main chain of the R-OH group is less than 8), a hydroxyl-containing thiazolium halide (X^{- +}Thi-R-OH, R-OH is a substituent group on the nitrogen atom of the thiazole ring, and the number of carbon atoms in the main chain of the R-OH group is less than 8), a hydroxyl-containing imidazolium halide catalyst with a long alkyl chain in the structural formula I (X^{- +}R'Im-R-OH, wherein the number of carbon atoms in the main chain of the R-OH group is 8 or more), or a hydroxyl-containing thiazolium halide catalyst with a long alkyl chain in the structural formula I (X^{- +}Thi-R-OH, wherein the number of carbon atoms in the main chain of the R-OH group is 8 or more);
(2) subjecting the hydroxyl-containing imidazolium halide, hydroxyl-containing thiazolium halide, hydroxyl-containing imidazolium halide catalyst with a long alkyl chain in the structural formula I or hydroxyl-containing thiazolium halide catalyst with a long alkyl chain in the structural formula I obtained in step (1) to a Lewis acid-base neutralization reaction with 1-3 times the molar amount of a Lewis acidic metal ion chloride (MClₙ), to convert the anion of the above salt from X⁻ to m·MClₙ-X⁻, thereby giving another type of hydroxyl-containing imidazolium salt (m·MClₙ-X^{- +}R'Im-R-OH, wherein the number of carbon atoms in the main chain of the R-OH group is less than 8), another type of hydroxyl-containing thiazolium salt (m·MClₙ-X^{- +}Thi-R-OH, wherein the number of carbon atoms in the main chain of the R-OH group is less than 8), another type of hydroxyl-containing imidazolium salt catalyst with a long alkyl chain in the structural formula I (m·MClₙ-X^{- +}R'Im-R-OH, wherein the number of carbon atoms in the main chain of the R-OH group is 8 or more) or another type of hydroxyl-containing thiazolium salt catalyst with a long alkyl chain in the structural formula I (m·MClₙ-X⁻⁺Thi-R-OH, wherein the number of carbon atoms in the main chain of the R-OH group is 8 or more);
(3) subjecting the other type of hydroxyl-containing imidazolium salt, the other type of hydroxyl-containing thiazolium salt, the other type of hydroxyl-containing imidazolium salt catalyst with a long alkyl chain in the structural formula I or the other type of hydroxyl-containing thiazolium salt catalyst with a long alkyl chain in the structural formula I obtained in step (2) to a ring-opening polymerization reaction with 1-100 times the molar amount of a cyclic ester or a cyclic ether-ester to give an imidazolium salt catalyst with a long-chain structure of polyester or polyether-ester in the structural formula I (m·MClₙ-X⁻⁺R'Im-R-poly(ester unit)ₙ-OH (ester unit refers to a structural unit of a polymer containing an ester group, and the subscript n refers to the degree of polymerization)) or a thiazolium salt catalyst with a long-chain structure of polyester or polyether-ester in the structural formula I (m·MClₙ-X^{- +}Thi-R-poly(ester unit)ₙ-OH); or mixing the other type of hydroxyl-containing imidazolium salt, the other type of hydroxyl-containing thiazolium salt, the other type of hydroxyl-containing imidazolium salt catalyst with a long alkyl chain in the structural formula I or the other type of hydroxyl-containing thiazolium salt catalyst with a long alkyl chain in the structural formula I obtained in step (2) with 1-100 times the molar amount of a hydroxy acid or with 1-100 times the molar amount of a dibasic acid and 1-100 times the molar amount of a diol to give an imidazolium salt or thiazolium salt catalyst with a long-chain structure of aliphatic polyester in the structural formula I through an esterification polycondensation reaction (m·MClₙ-X^{- +}R'Im-R-poly(ester unit)ₙ-OH or m·MClₙ-X^{- +}Thi-R-poly(ester unit)ₙ-OH),

the process steps and conditions of the preparation method two are as follows:
   (1) mixing a hydroxyl-containing organic carboxylic acid or a hydroxyl-containing organic sulfonic acid with an alkali metal hydroxide, an alkaline earth metal hydroxide or a quaternary ammonium base at a molar ratio of carboxylate or sulfonate to hydroxide of 0.8-1.2, and preparing a hydroxyl-containing organic carboxylate (wherein the number of carbon atoms in the main chain is less than 8), organic sulfonate (wherein the number of carbon atoms in the main chain is less than 8), a hydroxyl-containing organic carboxylate catalyst with a long alkyl chain in the structural formula II (the number of carbon atoms in the main chain is 8 or more) or a hydroxyl-containing organic sulfonate catalyst with a long alkyl chain in the structural formula II (the number of carbon atoms in the main chain is 8 or more) whose cation is an alkali metal ion, an alkaline earth metal ion or a quaternary ammonium ion through a Bronsted-Lowry acid-base neutralization reaction; or mixing a cyclic ester with an alkali metal hydroxide, an alkaline earth metal hydroxide or a quaternary ammonium base at a molar ratio of cyclic ester to hydroxide of 0.8-1.2, and preparing a hydroxyl-containing organic carboxylate (the number of carbon atoms in the main chain is less than 8) or a hydroxyl-containing organic sulfonate catalyst with a long alkyl chain in the structural formula II (the number of carbon atoms in the main chain is 8 or more) whose cation is an alkali metal ion, an alkaline earth metal ion or a quaternary ammonium ion through an ester bond alkaline hydrolysis reaction;
   (2) mixing the organic carboxylate, organic sulfonate, hydroxyl-containing organic carboxylate catalyst with a long alkyl chain in the structural formula II or hydroxyl-containing organic sulfonate catalyst with a long alkyl chain in the structural formula II whose cation is an alkali metal ion obtained in step (1) with a Lewis acidic metal ion chloride (MClₙ) at a molar ratio of carboxylate or sulfonate to chloride ion of 1-2, and preparing another type of hydroxyl-containing organic carboxylate (wherein the number of carbon atoms in the main chain is less than 8), organic sulfonate (wherein the number of carbon atoms in the main chain is less than 8), hydroxyl-containing organic carboxylate catalyst with a long alkyl chain in the structural formula II (wherein the number of carbon atoms in the main chain is 8 or more) or hydroxyl-containing organic sulfonate catalyst with a long alkyl chain in the structural formula II (wherein the number of carbon atoms in the main chain is 8 or more) whose cation is a Lewis acidic metal ion through a metathesis reaction;
   (3) mixing the hydroxyl-containing organic carboxylate, organic sulfonate, hydroxyl-containing organic carboxylate catalyst with a long alkyl chain in the structural formula II or hydroxyl-containing organic sulfonate catalyst with a long alkyl chain in the structural formula II whose cation is an alkali metal ion, an alkaline earth metal ion, a quaternary ammonium ion or a Lewis acidic metal ion obtained in step (2) with 1-100 times the molar amount of a cyclic ester or a cyclic ether-ester to give a catalyst with a long-chain structure of polyester or polyether-ester in the structural formula II through a ring-opening polymerization reaction, or with 1-100 times the molar amount of a hydroxy acid or 1-100 times the molar amount of a dibasic acid and 1-100 times the molar amount of a diol to give a catalyst with a long-chain structure of polyester in the structural formula II through an esterification polycondensation reaction,
the process steps and conditions of the preparation method three are as follows:
   (1) mixing an aliphatic polyester or polyether-ester with an alkali metal hydroxide, an alkaline earth metal hydroxide or a quaternary ammonium base at a molar ratio of ester bond to hydroxide of 1-100, and then performing an ester bond alkaline hydrolysis reaction at a temperature higher than the melting point of the corresponding polymer to give an aliphatic polyester or polyether-ester catalyst containing a terminal carboxylate and a terminal hydroxyl whose cation is an alkali metal ion, an alkaline earth metal ion or a quaternary ammonium ion in the structural structure II; or mixing a polyether containing a terminal carboxylic acid and a terminal hydroxyl with an alkali metal hydroxide, an alkaline earth metal hydroxide or a quaternary ammonium base at a molar ratio of terminal carboxylate to hydroxide of 0.8-1.2, and then performing a Bronsted-Lowry acid-base neutralization reaction at a temperature higher than the melting point of the corresponding polymer to give an aliphatic polyester, polyether-ester or polyether catalyst containing a terminal carboxylate and a terminal hydroxyl whose cation is an alkali metal ion, an alkaline earth metal ion or a quaternary ammonium ion in the structural formula II;
   (2) mixing the aliphatic polyester, polyether-ester or polyether catalyst containing a terminal carboxylate and a terminal hydroxyl whose cation is an alkali metal ion in the structural formula II obtained in step (1) with a Lewis acidic metal ion chloride (MClₙ) at a molar ratio of carboxylate to chloride ion of 0.8-2.2, and then performing a metathesis reaction at a temperature higher than the melting point of the corresponding polymer to give an aliphatic polyester, polyether-ester or polyether catalyst containing a terminal carboxylate and a terminal hydroxyl whose cation is a Lewis acidic metal ion in the structural formula II.

The hydroxyl-containing alkyl halide used in the quaternization reaction described in the above method one is alternatively 8-chloro-1-octanol, 3-chloro-1-propanol or 2-bromo-1-ethanol, and the imidazole or thiazole structure-containing compound used is alternatively 1-methylimidazole, 1-butylimidazole, 4-methylthiazole, thiazole or benzothiazole, the reaction temperature is alternatively 60-120 °C, yet alternatively 100-120 °C, the reaction time is alternatively 12-48 h, yet alternatively 12-24 h.

The MClₙ used in the Lewis acid-base neutralization reaction described in the above method one is alternatively zinc chloride (ZnCl₂), stannous chloride (SnCl₂), aluminum chloride (AlCl₃) or ferric chloride (FeCl₃), the reaction temperature is alternatively 25-120 °C, yet alternatively 25-80 °C, the reaction time is alternatively 0.5-12 h, yet alternatively 0.5-4 h.

The cyclic ester or cyclic ether-ester used in the ring-opening polymerization reaction described in the above method one or method two is alternatively lactide (LA), glycolide (GA), ε-caprolactone (ε-CL), δ-valerolactone (δ-VL), p-dioxanone (PDO), 3,4-dihydro-2H-benzo[1,4]dioxepine-2-one (BDXO) or 4-phenyl-3,4-dihydro-2H-benzo[1,4]dioxepine-2-one (BDXOP), the reaction temperature is alternatively 60-220 °C, yet alternatively 120-180 °C, the reaction time is alternatively 1-48 h, yet alternatively 2-12 h.

The dibasic acid used in the esterification polycondensation reaction described in the above method one or method two is alternatively 1,4-butanedioic acid or 1,6-hexanedioic acid, the diol used is alternatively 1,2-ethanediol, 1,4-butanediol or 1,6-hexanediol, the reaction temperature is alternatively 80-220 °C, yet alternatively 80-160 °C, the reaction time is alternatively 8-48 h, yet alternatively 8-16 h, the reaction pressure is alternatively 1-1*10⁴ Pa, yet alternatively 100-3000 Pa.

The hydroxyl-containing organic carboxylic acid used in the Bronsted-Lowry acid-base neutralization reaction described in the above method two is alternatively 12-hydroxyoctadecanoic acid, 6-hydroxyhexanoic acid, 18-hydroxyoctadecanoic acid or 8-hydroxyoctanoic acid, the hydroxyl-containing organic sulfonic acid used is 4-hydroxy-1-butanesulfonic acid, 3-hydroxy-1-propanesulfonic acid or 2-hydroxyethanesulfonic acid, the alkali metal hydroxide used is alternatively sodium hydroxide or potassium hydroxide, the alkaline earth metal hydroxide used is alternatively magnesium hydroxide or calcium hydroxide, the quaternary ammonium base used is alternatively tetramethylammonium hydroxide or tetraethylammonium hydroxide, the reaction temperature is alternatively 25-180 °C, yet alternatively 60-120 °C, the reaction time is alternatively 0.5-12 h, yet alternatively 0.5-8 h.

The cyclic ester used in the ester bond alkaline hydrolysis reaction described in the above method two is alternatively cyclopentadecanolide or ε-CL, the alkali metal hydroxide used is alternatively sodium hydroxide or potassium hydroxide, the alkaline earth metal hydroxide used is alternatively magnesium hydroxide or calcium hydroxide, the quaternary ammonium base used is alternatively tetramethylammonium hydroxide or tetraethylammonium hydroxide, the reaction temperature is alternatively 25-180 °C, yet alternatively 60-120 °C, the reaction time is 0.5-12 h, yet alternatively 0.5-8 h.

The MClₙ used in the metathesis reaction described in the above method two or method three is alternatively ZnCl₂, SnCl₂, AlCl₃ or FeCl₃, the reaction temperature is alternatively 25-220 °C, yet alternatively 120-220 °C, the reaction time is alternatively 0.5-12 h, yet alternatively 0.5-4 h.

The aliphatic polyester used in the ester bond alkaline hydrolysis reaction described in the above method three is alternatively PES, PBS, PHS, PEA, PBA, PHA, PLA, PGA, PCL or PVL, the polyether-ester used is alternatively PPDO, PBDXO or PDBXOP, the reaction temperature is alternatively 60-220 °C, yet alternatively 120-220 °C, the reaction time is 1-48 h, yet alternatively 2-12 h.

The polyether used in the Bronsted-Lowry acid-base neutralization reaction described in the above method three is alternatively PEG or PTHF, the reaction temperature is alternatively 25-120 °C, yet alternatively 25-80 °C, the reaction time is 0.5-12 h, yet alternatively 0.5-4 h.

The quaternization reaction, ring-opening polymerization reaction, esterification polycondensation reaction, Bronsted-Lowry acid-base neutralization reaction, ester bond alkaline hydrolysis reaction and metathesis reaction described in the above methods are all conventional classical reactions well known in the art.

Provided herein is use of the above catalyst for polyester depolymerization or cyclic ester synthesis, wherein the use is for catalyzing the hydrolysis, alcoholysis or cyclodepolymerization of a polyester homopolymer, copolymer, blend or complex to recover the corresponding monomers or monomers and oligomers, or for catalyzing the synthesis of a cyclic ester monomer or a cyclic ester monomer and cyclic oligomer from hydroxy acids or hydroxy esters through a polycondensation and cyclization reaction.

In the above use of the catalyst, the specific method of catalyzing the hydrolysis, alcoholysis or cyclodepolymerization of a polyester homopolymer, copolymer, blend or complex to recover the corresponding monomers or monomers and oligomers is as follows: mixing the polyester, water or alcohol, and catalyst according to a proportion, heating the mixture to 40-200 °C at 0.1-2 MPa for a hydrolysis or alcoholysis reaction for 1-12 h, and then recovering a solution of the corresponding monomers or monomers and oligomers in water or alcohol; or heating the mixture to 80-400 °C at 1-1*10⁵ Pa for cyclodepolymerization for 0.5-12 h, and simultaneously distilling or extracting the mixture to separate the generated cyclic ester monomers or cyclic ester monomers and cyclic oligomers from the reaction system; wherein the ratio of polyester to water or alcohol is 100:50-2000 wt%, and the molar ratio of the catalyst to the ester bond in the polyester is 0.01-10 mol% based on the terminal hydroxyl.

In the above specific method of catalyzing the recovery of corresponding monomers or oligomers, the ratio of water or alcohol added to the polyester in the hydrolysis or alcoholysis reaction is alternatively 100-500 wt%; the ratio of the added catalyst is alternatively 0.1-2 mol%; the pressure of the hydrolysis or alcoholysis reaction is alternatively 0.1-0.8 MPa, the temperature is alternatively 80-160 °C, and the time is alternatively 2-6 h; the pressure of the cyclodepolymerization reaction is alternatively 1-1000 Pa, the temperature is alternatively 120-220 °C, and the time is alternatively 0.5-2 h.

In the above specific method of catalyzing the recovery of corresponding monomers or oligomers, the polyester homopolymer, copolymer, blend or complex contains a structural unit of aliphatic polyester of a dibasic acid and a diol of the following structural formula i, a structural unit of polycyclic ester of the following structural formula ii, or a structural unit of polyether-ester: 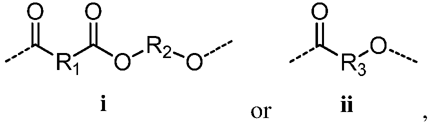 in the formulas, R₁ and R₂ are aliphatic groups, which may be the same or different, and R₃ is an aliphatic or aromatic group.

In the above specific method of catalyzing the recovery of corresponding monomers or oligomers, the corresponding monomers or monomers and oligomers recovered by hydrolyzing an aliphatic polyester of a dibasic acid and a diol are monomers of a dibasic acid of the following structural formula iii, monomers of a diol of the following structural formula iv, or oligomers of a dibasic acid and a diol of the following structural formula v; the corresponding monomers or monomers and oligomers recovered by alcoholysis are monomers of a diester of a dibasic acid of the following structural formula vi, monomers of a diol of the following structural formula iv, or oligomers of an ester of a dibasic acid and a diol of the following structural formula vii: 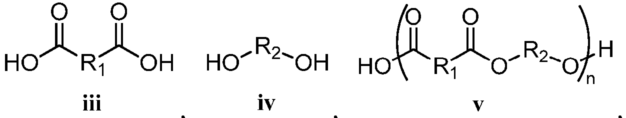 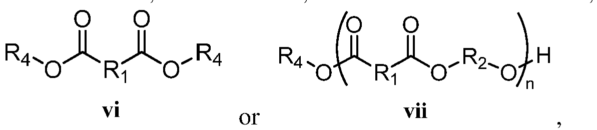 in the formulas, R₁ and R₂ are aliphatic groups, R₄ is methyl, ethyl, hydroxyethyl, hydroxybutyl or hydroxyhexyl, and the degree of polymerization n is 2 or 3.

In the above specific method of catalyzing the recovery of corresponding monomers or oligomers, the corresponding monomers or monomers and oligomers recovered by hydrolysis of a polycyclic ester or a polyether-ester are monomers of a hydroxy acid of the following structural formula viii, oligomers of a hydroxy acid of the following structural formula ix; the corresponding monomers or monomers and oligomers recovered by alcoholysis are monomers of a hydroxy ester of the following structural formula x, oligomers of a hydroxy ester of the following structural formula xi; the corresponding monomers or monomers and oligomers recovered by cyclodepolymerization are monomers of a cyclic ester or cyclic ether ester of the following structural formula xii, or cyclic oligomers of a cyclic ester or cyclic ether ester of the following structural formula xiii: 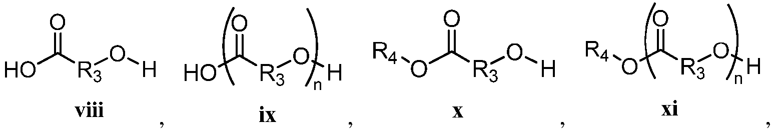 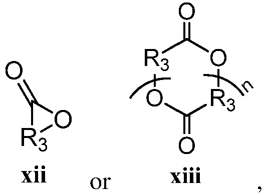 in the formulas, R₃ is an aliphatic or aromatic group, the degree of polymerization n in formula ix and formula xi is 2 or 3, and the degree of polymerization n in formula xiii is 1 or 2.

In the above specific method of catalyzing the polyester hydrolysis, alcoholysis or cyclodepolymerization to recover corresponding monomers or oligomers, the polyester with the structural unit of an aliphatic polyester of a dibasic acid and a diol is at least one of PES, PBS, PHS, PEA, PBA and PHA, the polyester with the structural unit of a polycyclic ester is at least one of PLA, PGA, PCL and PVL, and the polyester with the structural unit of a polyether ester is at least one of PPDO, PBDXO and PDBXOP.

In the use of the above catalyst, the specific method of catalyzing the synthesis of a cyclic ester monomer or a cyclic ester monomer and cyclic oligomer from hydroxy acids or hydroxy esters through a polycondensation and cyclization reaction is as follows:
(1) first, mixing the catalyst with a hydroxy acid or hydroxy ester at a molar ratio of 0.01-10 mol% based on the terminal hydroxyl, and then heating the mixture to 80-220 °C at 100-1*10⁵ Pa for a polycondensation reaction for 4-12 h, and simultaneously distilling the mixture to remove the water or alcohol generated in the reaction system to give an oligomer of the hydroxy acid;
(2) heating the obtained oligomer of hydroxy acid to 80-400 °C at 1-1*10⁵ Pa for a cyclodepolymerization reaction for 0.5-4 h, and simultaneously distilling or extracting the resulting mixture to separate the generated cyclic ester monomers or cyclic ester monomers and cyclic oligomers from the reaction system.

In the above method of catalyzing the synthesis of a cyclic ester monomer or a cyclic ester monomer and a cyclic oligomer, the hydroxy acid is alternatively glycolic acid, lactic acid, 6-hydroxyhexanoic acid, 5-hydroxypentanoic acid or 12-hydroxyoctadecanoic acid, and the hydroxy ester is any one of methyl glycolate, ethyl glycolate, methyl lactate, ethyl lactate, methyl 6-hydroxyhexanoate, ethyl 6-hydroxyhexanoate, methyl 5-hydroxypentanoate, ethyl 5-hydroxypentanoate, methyl 12-hydroxyoctadecanoate and ethyl 12-hydroxyoctadecanoate.

In the above method of catalyzing the synthesis of a cyclic ester monomer or a cyclic ester monomer and cyclic oligomer, the cyclic ester monomer and cyclic oligomer are glycolide, lactide, ε-caprolactone monomer or ε-caprolactone cyclic oligomer.

Compared with the prior art, the present disclosure has one or more of the following positive effects:
1. Since the catalyst provided herein is designed with a terminal hydroxyl structure and a long-chain structure, these structures can reduce the molecular weight of reactants by ester exchange with the polyester, thereby achieving the effect of reducing the viscosity of the reaction system, and avoiding the challenges to the equipment and process design caused by mass transfer problems.
2. Since the long-chain structure of the catalyst provided herein is completely compatible with a long-chain reactant polyester, the catalytic active center can be fully in contact with the reactant polyester, thereby greatly improving the utilization rate of the catalytic active center, reducing the consumption of the catalyst and improving the overall catalytic efficiency.
3. Since the composition of the long-chain structure of the catalyst provided herein is designable and selective, the composition of the long-chain structure can be adjusted to make it selectively compatible with a certain phase in the polyester blend or complex, thereby achieving selective depolymerization of the polyester blend or complex.
4. Since the catalyst provided herein is a long-chain catalyst, the difference in the molecular weight between the catalyst and the monomers and oligomers produced by depolymerization, and the difference in the physical properties between the catalyst and the product, such as boiling point and solubility, are huge. Therefore, it is easy to achieve the separation and purification of the product and recycling of the catalyst, thereby making the catalytic depolymerization method more environmentally friendly.
5. Since both the method of preparing the catalyst and the method of using the catalyst in polyester recovery and synthesis of cyclic ester monomers and cyclic oligomers provided herein are conventional classical reactions, the operation is simple and convenient for industrial production.

The following examples are provided to further illustrate the present disclosure in detail, but the following examples should not be understood as a limit to the scope of the present disclosure. Some non-essential improvements and adjustments made by the skilled in the art to the technical solutions based on the contents of this disclosure still fall within the scope of the present disclosure.

It is worth mentioning that, 1) in the catalysts obtained in examples 1 to 80, when the long-chain structure is a long alkyl chain, the number of carbon atoms in the long-chain structure = the number of carbon atoms in the R-OH group. 2) When the long-chain structure is a polyester chain, a polyether-ester chain or a polyether chain, the number of carbon atoms in the long-chain structure = the average degree of polymerization of the chain segment of the polymer * the number of carbon atoms of the main chain in the structural unit + the number of carbon atoms in the R-OH group. Among them, the calculation of the average degree of polymerization of the chain segment of the polymer is as shown in Fig. 3 and Fig. 5. In addition, the composition of the halogenated metal chloride m·MClₙ-X⁻ was verified by laser Raman spectrum (as shown in Fig. 1 and Fig. 2). 3) The hydrolysis or alcoholysis conversion rate obtained in application examples 1 to 117 = 1-(mass of the residual reactant/mass of the reactant)*100%. 4) The obtained cyclodepolymerization recovery rate = (mass of the recovered product/mass of the reactant) * 100%, the chemical structure of the obtained cyclodepolymerization product was verified by ¹H NMR (as shown in Fig. 7, Fig. 9, and Fig. 11), and the composition and purity of the product were measured by gas chromatography (as shown in Fig. 8, Fig. 10, and Fig. 12). 5) The melt viscosity of the cyclodepolymerization reaction system was measured in situ by an online viscometer (using a BROOKFIELD DV-II+ Pro online viscometer, equipped with an LV-1 model rotor, and measured at a rotational speed of 2.5-100 RPM).

### Example 1

(1) 40 mmol 1-methylimidazole and 40 mmol 8-chloro-1-octanol were added into a round-bottom flask. The mixture was heated, stirred and reacted at 100 °C in an oil bath under nitrogen for 48 h. Then, the mixture was washed with ethyl acetate and dried with baking to give 1-hydroxyoctyl-3-methylimidazolium chloride (Cl^{- +}MIm-C₈-OH). (2) 20 mmol Cl⁻⁺MIm-C₈-OH and 20 mmol FeCl₃ were added into a round-bottom flask. The mixture was heated, stirred and reacted at 80 °C in an oil bath under nitrogen for 0.5 h to give FeCl₃-Cl⁻⁺MIm-C₈-OH. (3) 10 mmol FeCl₃-Cl^{- +}MIm-C₈-OH and 100 mmol δ-VL were added into a round-bottom flask. The mixture was heated, stirred and reacted at 140 °C in an oil bath under nitrogen for 4 h to give FeCl₃-Cl⁻ *MIm-C₈-poly(δ-VL)₁₀-OH through ring-opening polymerization. The long-chain structure of the products of step (1) and step (2) was a long alkyl chain, and the number of carbon atoms in the main chain was 8; the long-chain structure of the product of step (3) was an aliphatic polyester chain, and the number of carbon atoms in the main chain was 5*10+8=58.

### Example 2

(1) 40 mmol 1-methylimidazole and 48 mmol 3-chloro-1-propanol were added into a round-bottom flask. The mixture was heated, stirred and reacted at 80 °C in an oil bath under nitrogen for 48 h. Then, the mixture was washed with ethyl acetate and dried with baking to give 1-hydroxypropyl-3-methylimidazolium chloride (Cl^{- +}MIm-C₃-OH). (2) 20 mmol Cl⁻⁺MIm-C₃-OH and 20 mmol FeCl₃ were added into a round-bottom flask. The mixture was heated, stirred and reacted at 25 °C in an oil bath under nitrogen for 4 h to give FeCl₃-Cl⁻⁺MIm-C₃-OH. (3) 10 mmol FeCl₃-Cl^{- +}MIm-C₃-OH and 50 mmol δ-VL were added into a round-bottom flask. The mixture was heated, stirred and reacted at 120 °C in an oil bath under nitrogen for 8 h to give FeCl₃-Cl^{- +}MIm-C₃-poly(δ-VL)₅-OH through ring-opening polymerization. The long-chain structure of the product of step (3) was an aliphatic polyester chain, and the number of carbon atoms in the main chain was 5*5+3 = 28.

### Example 3

(1) 40 mmol 1-methylimidazole and 32 mmol 2-bromo-1-ethanol were added into a round-bottom flask. The mixture was heated, stirred and reacted at 60 °C in an oil bath under nitrogen for 24 h. Then, the mixture was washed with ethyl acetate and dried with baking to give 1-hydroxyethyl-3-methylimidazolium bromide (Br^{- +}MIm-C₂-OH). (2) 20 mmol Br⁻⁺MIm-C₂-OH and 20 mmol FeCl₃ were added into a round-bottom flask. The mixture was heated, stirred and reacted at 25 °C in an oil bath under nitrogen for 12 h to give FeCl₃-Br⁻⁺MIm-C₂-OH. (3) 10 mmol FeCl₃-Br^{- +}MIm-C₂-OH and 300 mmol δ-VL were added into a round-bottom flask. The mixture was heated, stirred and reacted at 160 °C in an oil bath under nitrogen for 2 h to give FeCl₃-Br^{- +}MIm-C₂-poly(δ-VL)₃₀-OH through ring-opening polymerization. The long-chain structure of the product of step (3) was an aliphatic polyester chain, and the number of carbon atoms in the main chain was 5*30+2=152.

### Example 4

(1) 40 mmol 1-butylimidazole and 36 mmol 3-chloro-1-propanol were added into a round-bottom flask. The mixture was heated, stirred and reacted at 110 °C in an oil bath under nitrogen for 22 h. Then, the mixture was washed with ethyl acetate and dried with baking to give 1-hydroxypropyl-3-butylimidazolium chloride (Cl^{- +}BIm-C₃-OH). (2) 20 mmol Cl⁻⁺BIm-C₃-OH and 20 mmol FeCl₃ were added into a round-bottom flask. The mixture was heated, stirred and reacted at 40 °C in an oil bath under nitrogen for 6 h to give FeCl₃-Cl^{- +}BIm-C₃-OH. (3) 10 mmol FeCl₃-Cl^{- +}BIm-C₃-OH and 500 mmol δ-VL were added into a round-bottom flask. The mixture was heated, stirred and reacted at 100 °C in an oil bath under nitrogen for 24 h to give FeCl₃-Cl^{- +}BIm-C₃-poly(δ-VL)₅₀-OH through ring-opening polymerization. The long-chain structure of the product of step (3) was an aliphatic polyester chain, and the number of carbon atoms in the main chain was 5*50+3=253.

### Example 5

(1) 40 mmol 4-methylthiazole and 44 mmol 3-chloro-1-propanol were added into a round-bottom flask. The mixture was heated, stirred and reacted at 100 °C in an oil bath under nitrogen for 48 h. Then, the mixture was washed with ethyl acetate and dried with baking to give N-hydroxypropyl-4-methylthiazolium chloride (Cl^{- +}MThi-C₃-OH). (2) 20 mmol Cl⁻⁺MThi-C₃-OH and 20 mmol FeCl₃ were added into a round-bottom flask. The mixture was heated, stirred and reacted at 60 °C in an oil bath under nitrogen for 3 h to give FeCl₃-Cl⁻⁺MThi-C₃-OH. (3) 10 mmol FeCl₃-Cl^{- +}MThi-C₃-OH and 700 mmol δ-VL were added into a round-bottom flask. The mixture was heated, stirred and reacted at 180 °C in an oil bath under nitrogen for 4 h to give FeCl₃-Cl^{- +}MThi-C₃-poly(δ-VL)₇₀-OH through ring-opening polymerization. The long-chain structure of the product of step (3) was an aliphatic polyester chain, and the number of carbon atoms in the main chain was 5*70+3=353.

### Example 6

(1) 40 mmol thiazole and 40 mmol 3-chloro-1-propanol were added into a round-bottom flask. The mixture was heated, stirred and reacted at 90 °C in an oil bath under nitrogen for 36 h. Then, the mixture was washed with ethyl acetate and dried with baking to give N-hydroxypropylthiazolium chloride (Cl^{- +}Thi-C₃-OH). (2) 20 mmol Cl^{- +}Thi-C₃-OH and 20 mmol FeCl₃ were added into a round-bottom flask. The mixture was heated, stirred and reacted at 100 °C in an oil bath under nitrogen for 0.5 h to give FeCl₃-Cl^{- +}Thi-C₃-OH. (3) 10 mmol FeCl₃-Cl^{- +}Thi-C₃-OH and 900 mmol δ-VL were added into a round-bottom flask. The mixture was heated, stirred and reacted at 140 °C in an oil bath under nitrogen for 24 h to give FeCl₃-Cl⁻⁺Thi-C₃-poly(δ-VL)₉₀-OH through ring-opening polymerization. The long-chain structure of the product of step (3) was an aliphatic polyester chain, and the number of carbon atoms in the main chain was 5*90+3=453.

### Example 7

(1) 40 mmol benzothiazole and 40 mmol 3-chloro-1-propanol were added into a round-bottom flask. The mixture was heated, stirred and reacted at 120 °C in an oil bath under nitrogen for 12 h. Then, the mixture was washed with ethyl acetate and dried with baking to give N-hydroxypropylbenzothiazolium chloride (Cl^{- +}BzThi-C₃-OH). (2) 20 mmol Cl⁻⁺BzThi-C₃-OH and 20 mmol FeCl₃ were added into a round-bottom flask. The mixture was heated, stirred and reacted at 80 °C in an oil bath under nitrogen for 8 h to give FeCl₃-Cl⁻⁺BzThi-C₃-OH. (3) 10 mmol FeCl₃-Cl^{- +}BzThi-C₃-OH and 100 mmol δ-VL were added into a round-bottom flask. The mixture was heated, stirred and reacted at 140 °C in an oil bath under nitrogen for 4 h to give FeCl₃-Cl^{- +}BzThi-C₃-poly(δ-VL)₁₀-OH through ring-opening polymerization. The long-chain structure of the product of step (3) was an aliphatic polyester chain, and the number of carbon atoms in the main chain was 5*10+3=53.

### Example 8

(1) 40 mmol 1-methylimidazole and 40 mmol 3-chloro-1-propanol were added into a round-bottom flask. The mixture was heated, stirred and reacted at 100 °C in an oil bath under nitrogen for 48 h. Then, the mixture was washed with ethyl acetate and dried with baking to give 1-hydroxypropyl-3-methylimidazolium chloride (Cl^{- +}MIm-C₃-OH). (2) 20 mmol Cl⁻⁺MIm-C₃-OH and 20 mmol SnCl₂ were added into a round-bottom flask. The mixture was heated, stirred and reacted at 25 °C in an oil bath under nitrogen for 4 h to give SnCl₂-Cl⁻⁺MIm-C₃-OH. (3) 10 mmol SnCl₂-Cl^{- +}MIm-C₃-OH and 100 mmol δ-VL were added into a round-bottom flask. The mixture was heated, stirred and reacted at 140 °C in an oil bath under nitrogen for 3 h to give SnCl₂-Cl^{- +}MIm-C₃-poly(δ-VL)₁₀-OH through ring-opening polymerization. The long-chain structure of the product of step (3) was an aliphatic polyester chain, and the number of carbon atoms in the main chain was 5*10+3=53.

### Example 9

Step (1) was the same as example 8 (1). (2) 20 mmol Cl^{- +}MIm-C₃-OH and 20 mmol ZnCl₂ were added into a round-bottom flask. The mixture was heated, stirred and reacted at 25 °C in an oil bath under nitrogen for 4 h to give ZnCl₂-Cl^{- +}MIm-C₃-OH. (3) 10 mmol ZnCl₂-Cl⁻⁺MIm-C₃-OH and 100 mmol δ-VL were added into a round-bottom flask. The mixture was heated, stirred and reacted at 140 °C in an oil bath under nitrogen for 4 h to give ZnCl₂-Cl⁻⁺MIm-C₃-poly(δ-VL)₁₀-OH through ring-opening polymerization. The long-chain structure of the product of step (3) was an aliphatic polyester chain, and the number of carbon atoms in the main chain was 5*10+3=53.

### Example 10

Step (1) was the same as example 8 (1). (2) 20 mmol Cl^{- +}MIm-C₃-OH and 20 mmol AlCl₃ were added into a round-bottom flask. The mixture was heated, stirred and reacted at 25 °C in an oil bath under nitrogen for 4 h to give AlCl₃-Cl^{- +}MIm-C₃-OH. (3) 10 mmol AlCl₃-Cl⁻⁺MIm-C₃-OH and 100 mmol δ-VL were added into a round-bottom flask. The mixture was heated, stirred and reacted at 140 °C in an oil bath under nitrogen for 16 h to give AlCl₃-Cl⁻⁺MIm-C₃-poly(δ-VL)₁₀-OH through ring-opening polymerization. The long-chain structure of the product of step (3) was an aliphatic polyester chain, and the number of carbon atoms in the main chain was 5*10+3=53.

### Example 11

Step (1) was the same as example 8 (1). (2) 20 mmol Cl^{- +}MIm-C₃-OH and 34 mmol FeCl₃ were added into a round-bottom flask. The mixture was heated, stirred and reacted at 120 °C in an oil bath under nitrogen for 2 h to give 1.7·FeCl₃-Cl^{- +}MIm-C₃-OH. (3) 10 mmol 1.7·FeCl₃-Cl^{- +}MIm-C₃-OH and 100 mmol δ-VL were added into a round-bottom flask. The mixture was heated, stirred and reacted at 140 °C in an oil bath under nitrogen for 4 h to give 1.7·FeCl₃-Cl^{- +}MIm-C₃-poly(δ-VL)₁₀-OH through ring-opening polymerization. The long-chain structure of the product of step (3) was an aliphatic polyester chain, and the number of carbon atoms in the main chain was 5*10+3=53.

### Example 12

Step (1) was the same as example 8 (1). (2) 20 mmol Cl^{- +}MIm-C₃-OH and 60 mmol FeCl₃ were added into a round-bottom flask. The mixture was heated, stirred and reacted at 120 °C in an oil bath under nitrogen for 1 h to give 3·FeCl₃-Cl^{- +}MIm-C₃-OH. (3) 10 mmol 3·FeCl₃-Cl^{- +}MIm-C₃-OH and 100 mmol δ-VL were added into a round-bottom flask. The mixture was heated, stirred and reacted at 140 °C in an oil bath under nitrogen for 4 h to give 3·FeCl₃-Cl^{- +}MIm-C₃-poly(δ-VL)₁₀-OH through ring-opening polymerization. The long-chain structure of the product of step (3) was an aliphatic polyester chain, and the number of carbon atoms in the main chain was 5*10+3=53.

### Example 13

Step (1) was the same as example 8 (1). (2) 20 mmol Cl^{- +}MIm-C₃-OH and 26 mmol FeCl₃ were added into a round-bottom flask. The mixture was heated, stirred and reacted at 25 °C in an oil bath under nitrogen for 4 h to give 1.3·FeCl₃-Cl^{- +}MIm-C₃-OH. (3) 10 mmol 1.3·FeCl₃-Cl^{- +}MIm-C₃-OH and 100 mmol δ-VL were added into a round-bottom flask. The mixture was heated, stirred and reacted at 140 °C in an oil bath under nitrogen for 4 h to give 1.3·FeCl₃-Cl^{- +}MIm-C₃-poly(δ-VL)₁₀-OH through ring-opening polymerization. The long-chain structure of the product of step (3) was an aliphatic polyester chain, and the number of carbon atoms in the main chain was 5*10+3=53.

### Example 14

Step (1) was the same as example 8 (1). (2) 20 mmol Cl^{- +}MIm-C₃-OH and 20 mmol FeCl₃ were added into a round-bottom flask. The mixture was heated, stirred and reacted at 25 °C in an oil bath under nitrogen for 4 h to give FeCl₃-Cl^{- +}MIm-C₃-OH. (3) 10 mmol FeCl₃-Cl⁻⁺MIm-C₃-OH and 100 mmol L-lactide (L-LA) were added into a round-bottom flask. The mixture was heated, stirred and reacted at 140 °C in an oil bath under nitrogen for 4 h to give FeCl₃-Cl^{- +}MIm-C₃-poly(L-LA)₁₀-OH through ring-opening polymerization. The long-chain structure of the product of step (3) was an aliphatic polyester chain, and the number of carbon atoms in the main chain was 4*10+3=43.

### Example 15

Step (1) and step (2) were the same as example 14 (1) and (2). (3) 10 mmol FeCl₃-Cl ⁺MIm-C₃-OH and 100 mmol GA were added into a round-bottom flask. The mixture was heated and stirred well at 220 °C in an oil bath under nitrogen, and then subjected to a ring-opening polymerization reaction for 1 h to give FeCl₃-Cl^{- +}MIm-C₃-poly(GA)₁₀-OH. The long-chain structure of the product of step (3) was an aliphatic polyester chain, and the number of carbon atoms in the main chain was 4*10+3=43.

### Example 16

Step (1) and step (2) were the same as example 14 (1) and (2). (3) 10 mmol FeCl₃-Cl⁻⁺MIm-C₃-OH and 100 mmol ε-CL were added into a round-bottom flask. The mixture was heated, stirred and reacted at 140 °C in an oil bath under nitrogen for 12 h to give FeCl₃-Cl⁻⁺MIm-C₃-poly(ε-CL)₁₀-OH through ring-opening polymerization. The long-chain structure of the product of step (3) was an aliphatic polyester chain, and the number of carbon atoms in the main chain was 6*10+3=63.

### Example 17

Step (1) and step (2) were the same as example 14 (1) and (2). (3) 10 mmol FeCl₃-Cl ⁺MIm-C₃-OH and 100 mmol PDO were added into a round-bottom flask. The mixture was heated, stirred and reacted at 120 °C in an oil bath under nitrogen for 4 h, then cooled to 60 °C and reacted for 16 h to give FeCl₃-Cl^{- +}MIm-C₃-poly(PDO)₁₀-OH through ring-opening polymerization. The long-chain structure of the product of step (3) was a polyether-ester chain, and the number of carbon atoms in the main chain was 4*10+3=43.

### Example 18

Step (1) and step (2) were the same as example 14 (1) and (2). (3) 10 mmol FeCl₃-Cl⁻⁺MIm-C₃-OH and 100 mmol PDO were added into a round-bottom flask. The mixture was heated, stirred and reacted at 80 °C in an oil bath under nitrogen for 24 h to give FeCl₃-Cl⁻⁺MIm-C₃-poly(PDO)₁₀-OH through ring-opening polymerization. The long-chain structure of the product of step (3) was a polyether-ester chain, and the number of carbon atoms in the main chain was 4*10+3=43.

### Example 19

Step (1) and step (2) were the same as example 14 (1) and (2). (3) 10 mmol FeCl₃-Cl⁻⁺MIm-C₃-OH and 100 mmol BDXO were added into a round-bottom flask. The mixture was heated, stirred and reacted at 140 °C in an oil bath under nitrogen for 2 h, then cooled to 80 °C and reacted for 14 h to give FeCl₃-Cl^{- +}MIm-C₃-poly(BDXO)₁₀-OH through ring-opening polymerization. The long-chain structure of the product of step (3) was a polyether-ester chain, and the number of carbon atoms in the main chain was 5*10+3=53.

### Example 20

Step (1) and step (2) were the same as example 14 (1) and (2). (3) 10 mmol FeCl₃-Cl⁻⁺MIm-C₃-OH and 100 mmol BDXOP were added into a round-bottom flask. The mixture was heated, stirred and reacted at 140 °C in an oil bath under nitrogen for 2 h, then cooled to 80 °C and reacted for 14 h to give FeCl₃-Cl^{- +}MIm-C₃-poly(BDXOP)₁₀-OH through ring-opening polymerization. The long-chain structure of the product of step (3) was a polyether-ester chain, and the number of carbon atoms in the main chain was 5*10+3=53.

### Example 21

Step (1) and step (2) were the same as example 14 (1) and (2). (3) 10 mmol FeCl₃-Cl⁻⁺MIm-C₃-OH and 10 mmol δ-VL were added into a round-bottom flask. The mixture was heated, stirred and reacted at 140 °C in an oil bath under nitrogen for 1 h to give FeCl₃-Cl⁻⁺MIm-C₃-poly(δ-VL)₁-OH through ring-opening polymerization. The long-chain structure of the product of step (3) was an aliphatic polyester chain, and the number of carbon atoms in the main chain was 5*1+3=8.

### Example 22

Step (1) and step (2) were the same as example 14 (1) and (2). (3) 10 mmol FeCl₃-Cl⁻⁺MIm-C₃-OH and 1000 mmol δ-VL were added into a round-bottom flask. The mixture was heated, stirred and reacted at 140 °C in an oil bath under nitrogen for 48 h to give FeCl₃-Cl⁻⁺MIm-C₃-poly(δ-VL)₁₀₀-OH through ring-opening polymerization. The long-chain structure of the product of step (3) was an aliphatic polyester chain, and the number of carbon atoms in the main chain was 5*100+3=503.

### Example 23

Step (1) and step (2) were the same as example 14 (1) and (2). (3) 10 mmol FeCl₃-Cl⁻⁺MIm-C₃-OH and 200 mmol L-lactic acid were added into a round-bottom flask. The mixture was reacted successively at a controlled temperature of 80, 100, 120, and 140 °C in an oil bath for 2 h, respectively, at a pressure of 3000 Pa reduced by a water pump. Then, the mixture was reacted at 140 °C and a pressure of 100 Pa reduced by an oil pump for 8 h to give FeCl₃-Cl⁻⁺MIm-C₃-poly(L-LA)₁₀-OH through polycondensation. The long-chain structure of the product of step (3) was an aliphatic polyester chain, and the number of carbon atoms in the main chain was 4*10+3=43.

### Example 24

Step (1) and step (2) were the same as example 14 (1) and (2). (3) 10 mmol FeCl₃-Cl⁻⁺MIm-C₃-OH and 500 mmol glycolic acid were added into a round-bottom flask. The mixture was reacted successively at a controlled temperature of 80, 100, 120, and 140 °C in an oil bath for 2 h, respectively, at a pressure of 3000 Pa reduced by a water pump. Then, the mixture was reacted at 160 °C and a pressure of 100 Pa reduced by an oil pump for 6 h to give FeCl₃-Cl⁻⁺MIm-C₃-poly(GA)₂₅-OH through polycondensation. The long-chain structure of the product of step (3) was an aliphatic polyester chain, and the number of carbon atoms in the main chain was 4*25+3=103.

### Example 25

Step (1) and step (2) were the same as example 14 (1) and (2). (3) 10 mmol FeCl₃-Cl⁻⁺MIm-C₃-OH, 10 mmol succinic acid and 10 mmol ethylene glycol were added into a round-bottom flask. The mixture was reacted successively at a controlled temperature of 80, 100, 120, and 140 °C in an oil bath for 2 h, respectively, at a pressure of 3000 Pa reduced by a water pump to give FeCl₃-Cl^{- +}MIm-C₃-poly(ES)₁-OH through polycondensation. The long-chain structure of the product of step (3) was an aliphatic polyester chain, and the number of carbon atoms in the main chain was 6*1+3=9.

### Example 26

Step (1) and step (2) were the same as example 14 (1) and (2). (3) 10 mmol FeCl₃-Cl ⁺MIm-C₃-OH, 100 mmol succinic acid and 100 mmol butanediol were added into a round-bottom flask. The mixture was reacted successively at a controlled temperature of 80, 100, 120, and 140 °C in an oil bath for 2 h, respectively, at a pressure of 3000 Pa reduced by a water pump. Then, the mixture was reacted at 160 °C and a pressure of 100 Pa reduced by an oil pump for 8 h to give FeCl₃-Cl^{- +}MIm-C₃-poly(BS)₁₀-OH through polycondensation. The long-chain structure of the product of step (3) was an aliphatic polyester chain, and the number of carbon atoms in the main chain was 8*10+3=83.

### Example 27

Step (1) and step (2) were the same as example 14 (1) and (2). (3) 10 mmol FeCl₃-Cl⁻⁺MIm-C₃-OH, 700 mmol succinic acid and 700 mmol hexanediol were added into a round-bottom flask. The mixture was reacted successively at a controlled temperature of 80, 100, 120, and 140 °C in an oil bath for 2 h, respectively, at a pressure of 3000 Pa reduced by a water pump. Then, the mixture was reacted at 160 °C and a pressure of 100 Pa reduced by an oil pump for 40 h to give FeCl₃-Cl^{- +}MIm-C₃-poly(HS)₇₀-OH through polycondensation. The long-chain structure of the product of step (3) was an aliphatic polyester chain, and the number of carbon atoms in the main chain was 10*100+3=703.

### Example 28

Step (1) and step (2) were the same as example 14 (1) and (2). (3) 10 mmol FeCl₃-Cl⁻⁺MIm-C₃-OH, 100 mmol adipic acid and 100 mmol ethylene glycol were added into a round-bottom flask. The mixture was reacted successively at a controlled temperature of 120, 140, 160, and 180 °C in an oil bath for 2 h, respectively, at a pressure of 3000 Pa reduced by a water pump. Then, the mixture was reacted at 220 °C and a pressure of 100 Pa reduced by an oil pump for 2 h to give FeCl₃-Cl^{- +}MIm-C₃-poly(EA)₁₀-OH through polycondensation. The long-chain structure of the product of step (3) was an aliphatic polyester chain, and the number of carbon atoms in the main chain was 8*10+3=83.

### Example 29

Step (1) and step (2) were the same as example 14 (1) and (2). (3) 10 mmol FeCl₃-Cl⁻⁺MIm-C₃-OH, 100 mmol adipic acid and 100 mmol butanediol were added into a round-bottom flask. The mixture was reacted successively at a controlled temperature of 80, 100, 120, and 140 °C in an oil bath for 2 h, respectively, at a pressure of 1*10⁴ Pa reduced by a water pump. Then, the mixture was reacted at 160 °C and a pressure of 100 Pa reduced by an oil pump for 12 h to give FeCl₃-Cl^{- +}MIm-C₃-poly(BA)₁₀-OH through polycondensation. The long-chain structure of the product of step (3) was an aliphatic polyester chain, and the number of carbon atoms in the main chain was 10*10+3=103.

### Example 30

Step (1) and step (2) were the same as example 14 (1) and (2). (3) 10 mmol FeCl₃-Cl⁻⁺MIm-C₃-OH, 1000 mmol adipic acid and 1000 mmol hexanediol were added into a round-bottom flask. The mixture was reacted successively at a controlled temperature of 80, 100, 120, and 140 °C in an oil bath for 2 h, respectively, at a a pressure of 3000 Pa reduced by a water pump. Then, the mixture was reacted at 160 °C and a pressure of 1 Pa reduced by an oil pump for 40 h to give FeCl₃-Cl^{- +}MIm-C₃-poly(HA)₁₀₀-OH through polycondensation. The long-chain structure of the product of step (3) was an aliphatic polyester chain, and the number of carbon atoms in the main chain was 12*100+3=1203.

### Example 31

(1) 30 mmol 12-hydroxyoctadecanoic acid and 30 mmol sodium hydroxide were mixed in 50 mL of water, and the mixture was stirred at 90 °C for 0.5 h to give an aqueous solution of sodium 12-hydroxyoctadecanoate. (2) Then, 10 mmol AlCl₃ was added, and the mixture was stirred at 90 °C for another 0.5 h. Then, the mixture was cooled to room temperature, and the solid components were separated, collected, and dried with baking to give aluminum 12-hydroxyoctadecanoate (Al³⁺ [⁻OOC-C₁₁(C₆)-OH]₃). (3) 10 mmol Al³⁺ [⁻OOC-C₁₁(C₆)-OH]₃ and 300 mmol δ-VL were mixed in a round-bottom flask. The mixture was heated, stirred and reacted at 140 °C in an oil bath under nitrogen for 4 h to give Al³⁺ [⁻ OOC-C₁₁(C₆)-poly(δ-VL)₁₀-OH]₃ through ring-opening polymerization. The long-chain structure of the product of step (2) was a long alkyl chain, and the number of carbon atoms in the main chain was 12; the long-chain structure of the product of step (3) was an aliphatic polyester chain, and the number of carbon atoms in the main chain was 5*10+12=62.

### Example 32

(1) 30 mmol 6-hydroxyhexanoic acid and 30 mmol sodium hydroxide were mixed in 50 mL of water, and the mixture was stirred at 25 °C for 2 h to give an aqueous solution of sodium 6-hydroxyhexanoate. (2) Then, 10 mmol AlCl₃ was added, and the mixture was stirred for another 2 h. Then, the mixture was rotary evaporated to remove the water in the system, and dried with baking to give aluminum 6-hydroxyhexanoate (Al³⁺ (⁻OOC-C₅-OH)₃). (3) 10 mmol Al³⁺ (⁻OOC-C₅-OH)₃ and 150 mmol δ-VL were mixed in a round-bottom flask. The mixture was heated, stirred and reacted at 120 °C in an oil bath under nitrogen for 8 h to give Al³⁺ [⁻ OOC-C₅-poly(δ-VL)₅-OH]₃ through ring-opening polymerization. The long-chain structure of the product of step (3) was an aliphatic polyester chain, and the number of carbon atoms in the main chain was 5*5+6=31.

### Example 33

(1) 30 mmol 4-hydroxy-1-butanesulfonic acid and 24 mmol tetramethylammonium hydroxide were mixed in 50 mL of water, and the mixture was stirred at 60 °C for 12 h to give an aqueous solution of tetramethylammonium 4-hydroxybutanesulfonate; the solution was rotary evaporated to remove the water in the system, and dried with baking to give tetramethylammonium 4-hydroxybutanesulfonate (Me₄N^{+ -}SO₃-C₄-OH). (2) Then, 900 mmol δ-VL was added. The mixture was heated, stirred and reacted at 160 °C in an oil bath under nitrogen for 2 h to give Me₄N^{+ -}SO₃-C₄-poly(δ-VL)₃₀-OH through ring-opening polymerization. The long-chain structure of the product of step (2) was an aliphatic polyester chain, and the number of carbon atoms in the main chain was 5*30+4=154.

### Example 34

(1) 30 mmol 3-hydroxy-1-propanesulfonic acid and 30 mmol tetraethylammonium hydroxide were mixed in 50 mL of water, and the mixture was stirred at 100 °C for 8 h to give an aqueous solution of tetraethylammonium 3-hydroxypropanesulfonate; the solution was rotary evaporated to remove the water in the system, and dried with baking to give tetraethylammonium 4-hydroxybutanesulfonate (Et₄N^{+ -}SO₃-C₄-OH). (2) Then, 1500 mmol δ-VL was added. The mixture was heated, stirred and reacted at 100 °C in an oil bath under nitrogen for 24 h to give Et₄N^{+ -}SO₃-C₃-poly(δ-VL)₅₀-OH through ring-opening polymerization. The long-chain structure of the product of step (2) was an aliphatic polyester chain, and the number of carbon atoms in the main chain was 5*50+3=253.

### Example 35

(1) 30 mmol cyclopentadecalactone and 36 mmol sodium hydroxide were mixed, and the mixture was stirred at 120 °C for 12 h to give sodium 15-hydroxypentadecanoate. (2) Then, 12 mmol AlCl₃ and 50 mL of water were added, and the mixture was stirred at 100 °C for another 3 h. Then, the mixture was cooled to room temperature, and the solid components were separated, collected, and dried with baking to give aluminum 15-hydroxypentadecanoate (Al³⁺ (⁻ OOC-C₁₄-OH)₃). (3) 10 mmol Al³⁺ (⁻OOC-C₁₄-OH)₃ and 2100 mmol δ-VL were mixed in a round-bottom flask. The mixture was heated, stirred and reacted at 180 °C in an oil bath under nitrogen for 4 h to give Al³⁺ [⁻OOC-C₁₄-poly(δ-VL)₇₀-OH]₃ through ring-opening polymerization. The long-chain structure of the product of step (2) was a long alkyl chain, and the number of carbon atoms in the main chain was 15; the long-chain structure of the product of step (3) was an aliphatic polyester chain, and the number of carbon atoms in the main chain was 5*70+15=365.

### Example 36

(1) 30 mmol ε-caprolactone and 30 mmol potassium hydroxide were mixed, and the mixture was stirred at 140 °C for 8 h to give potassium 6-hydroxyhexanoate. (2) Then, 10 mmol AlCl₃ was added, and the mixture was stirred for another 0.5 h to give aluminum 6-hydroxyhexanoate (Al³⁺ (⁻OOC-C₅-OH)₃). (3) 10 mmol Al³⁺ (⁻OOC-C₅-OH)₃ and 2700 mmol δ-VL were mixed in a round-bottom flask. The mixture was heated, stirred and reacted at 140 °C in an oil bath under nitrogen for 24 h to give Al³⁺ [⁻OOC-C₅-poly(δ-VL)₉₀-OH]₃ through ring-opening polymerization. The long-chain structure of the products of step (1) and step (2) was a long alkyl chain, and the number of carbon atoms in the main chain was 15; the long-chain structure of the product of step (3) was an aliphatic polyester chain, and the number of carbon atoms in the main chain was 5*90+6=456.

### Example 37

(1) 40 mmol cyclopentadecalactone and 18 mmol magnesium hydroxide were mixed, and the mixture was stirred at 160 °C under nitrogen for 5 h to give magnesium 15-hydroxypentadecanoate (Mg²⁺ (⁻OOC-C₁₄-OH)₂). (2) 10 mmol Mg²⁺ (⁻OOC-C₁₄-OH)₂ and 200 mmol δ-VL were mixed in a round-bottom flask. The mixture was heated, stirred and reacted at 140 °C in an oil bath under nitrogen for 4 h to give Mg²⁺ [⁻ OOC-C₁₄-poly(δ-VL)₁₀-OH]₂ through ring-opening polymerization. The long-chain structure of the product of step (1) was a long alkyl chain, and the number of carbon atoms in the main chain was 15; the long-chain structure of the product of step (2) was an aliphatic polyester chain, and the number of carbon atoms in the main chain was 5*10+15=65.

### Example 38

(1) 40 mmol cyclopentadecalactone and 22 mmol calcium hydroxide were mixed, and the mixture was stirred at 180 °C under nitrogen for 1 h to give calcium 15-hydroxypentadecanoate (Ca²⁺ (⁻OOC-C₁₄-OH)₂). (2) 10 mmol Ca²⁺ (⁻OOC-C₁₄-OH)₂ and 200 mmol δ-VL were mixed in a round-bottom flask. The mixture was heated, stirred and reacted at 140 °C in an oil bath under nitrogen for 3 h to give Ca²⁺ [⁻ OOC-C₁₄-poly(δ-VL)₁₀-OH]₂ through ring-opening polymerization. The long-chain structure of the product of step (1) was a long alkyl chain, and the number of carbon atoms in the main chain was 15; the long-chain structure of the product of step (2) was an aliphatic polyester chain, and the number of carbon atoms in the main chain was 5*10+15=65.

### Example 39

(1) 30 mmol cyclopentadecalactone and 30 mmol sodium hydroxide were mixed, and the mixture was stirred at 120 °C for 8 h to give sodium 15-hydroxypentadecanoate. (2) Then, 10 mmol FeCl₃ was added, and the mixture was stirred at 140 °C for another 12 h. Then, the mixture was cooled to room temperature to give Fe³⁺ (⁻OOC-C₁₄-OH)₃. (3) 10 mmol Fe³⁺ (⁻ OOC-C₁₄-OH)₃ and 300 mmol δ-VL were mixed in a round-bottom flask. The mixture was heated, stirred and reacted at 140 °C in an oil bath under nitrogen for 4 h to give Fe³⁺ [⁻ OOC-C₁₄-poly(δ-VL)₁₀-OH]₃ through ring-opening polymerization. The long-chain structure of the products of step (1) and step (2) was a long alkyl chain, and the number of carbon atoms in the main chain was 15; the long-chain structure of the product of step (3) was an aliphatic polyester chain, and the number of carbon atoms in the main chain was 5*10+15=65.

### Example 40

Step (1) was the same as example 39 (1). (2) Then, 15 mmol ZnCl₂ was added, and the mixture was stirred at 160 °C for another 4 h. Then, the mixture was cooled to room temperature to give Zn²⁺ (⁻OOC-C₁₄-OH)₂. (3) 10 mmol Zn²⁺ (⁻OOC-C₁₄-OH)₂ and 200 mmol δ-VL were mixed in a round-bottom flask. The mixture was heated, stirred and reacted at 140 °C in an oil bath under nitrogen for 4 h to give Zn²⁺ [⁻OOC-C₁₄-poly(δ-VL)₁₀-OH]₂ through ring-opening polymerization. The long-chain structure of the products of step (1) and step (2) was a long alkyl chain, and the number of carbon atoms in the main chain was 15; the long-chain structure of the product of step (3) was an aliphatic polyester chain, and the number of carbon atoms in the main chain was 5*10+15=65.

### Example 41

Step (1) was the same as example 39 (1). (2) Then, 15 mmol SnCl₂ was added, and the mixture was stirred at 120 °C for another 1 h. Then, the mixture was cooled to room temperature to give Sn²⁺ (⁻OOC-C₁₄-OH)₂. (3) 10 mmol Sn²⁺ (⁻OOC-C₁₄-OH)₂ and 200 mmol δ-VL were mixed in a round-bottom flask. The mixture was heated, stirred and reacted at 140 °C in an oil bath under nitrogen for 4 h to give Sn²⁺ [⁻OOC-C₁₄-poly(δ-VL)₁₀-OH]₂ through ring-opening polymerization. The long-chain structure of the products of step (1) and step (2) was a long alkyl chain, and the number of carbon atoms in the main chain was 15; the long-chain structure of the product of step (3) was an aliphatic polyester chain, and the number of carbon atoms in the main chain was 5*10+15=65.

### Example 42

Step (1) was the same as example 39 (1). (2) Then, 20 mmol AlCl₃ was added, and the mixture was stirred at 220 °C for another 0.5 h. Then, the mixture was cooled to room temperature to give Al³⁺ (⁻OOC-C₁₄-OH)₃. (3) 10 mmol Al³⁺ (⁻OOC-C₁₄-OH)₃ and 300 mmol δ-VL were mixed in a round-bottom flask. The mixture was heated, stirred and reacted at 140 °C in an oil bath under nitrogen for 4 h to give Al³⁺ [⁻OOC-C₁₄-poly(δ-VL)₁₀-OH]₃ through ring-opening polymerization. The long-chain structure of the products of step (1) and step (2) was a long alkyl chain, and the number of carbon atoms in the main chain was 15; the long-chain structure of the product of step (3) was an aliphatic polyester chain, and the number of carbon atoms in the main chain was 5*10+15=65.

### Example 43

Step (1) was the same as example 39 (1). (2) Then, 16 mmol AlCl₃ was added, and the mixture was stirred at 180 °C for another 0.5 h. Then, the mixture was cooled to room temperature, and the solid components were separated and collected to give Al³⁺ (⁻ OOC-C₁₄-OH)₃. (3) 10 mmol Al³⁺ (⁻OOC-C₁₄-OH)₃ and 300 mmol δ-VL were mixed in a round-bottom flask. The mixture was heated, stirred and reacted at 140 °C in an oil bath under nitrogen for 4 h to give Al³⁺ [⁻OOC-C₁₄-poly(δ-VL)₁₀-OH]₃ through ring-opening polymerization. The long-chain structure of the products of step (1) and step (2) was a long alkyl chain, and the number of carbon atoms in the main chain was 15; the long-chain structure of the product of step (3) was an aliphatic polyester chain, and the number of carbon atoms in the main chain was 5*10+15=65.

### Example 44

Step (1) and step (2) were the same as example 31 (1) and (2). (3) 10 mmol Al³⁺ [⁻ OOC-C₁₁(C₆)-OH]₃ and 300 mmol L-LA were mixed in a round-bottom flask. The mixture was heated and reacted at 140 °C in an oil bath under nitrogen for 4 h to give Al³⁺ [⁻ OOC-C₁₁(C₆)-poly(L-LA)₁₀-OH]₃ through ring-opening polymerization. The long-chain structure of the product of step (3) was an aliphatic polyester chain, and the number of carbon atoms in the main chain was 4*10+12=52.

### Example 45

Step (1) and step (2) were the same as example 31 (1) and (2). (3) 10 mmol Al³⁺ [⁻ OO0-C₁₁(C₆)-OH]₃ and 300 mmol GA were mixed in a round-bottom flask. The mixture was heated and reacted at 220 °C in an oil bath under nitrogen for 1 h to give Al³⁺ [⁻ OOC-C₁₁(C₆)-poly(GA)₁₀-OH]₃ through ring-opening polymerization. The long-chain structure of the product of step (3) was an aliphatic polyester chain, and the number of carbon atoms in the main chain was 4*10+12=52.

### Example 46

Step (1) and step (2) were the same as example 31 (1) and (2). (3) 10 mmol Al³⁺ [⁻ OO0-C₁₁(C₆)-OH]₃ and 300 mmol ε-CL were mixed in a round-bottom flask. The mixture was heated and reacted at 140 °C in an oil bath under nitrogen for 12 h to give Al³⁺ [⁻ OOC-C₁₁(C₆)-poly(ε-CL)₁₀-OH]₃ through ring-opening polymerization. The long-chain structure of the product of step (3) was an aliphatic polyester chain, and the number of carbon atoms in the main chain was 6*10+12=72.

### Example 47

(1) 30 mmol 18-hydroxyoctadecanoic acid and 30 mmol sodium hydroxide were mixed in 50 mL of water, and the mixture was stirred at 90 °C for 0.5 h to give an aqueous solution of sodium 18-hydroxyoctadecanoate. (2) Then, 10 mmol AlCl₃ was added, and the mixture was stirred at 90 °C for another 0.5 h. Then, the mixture was cooled to room temperature, and the solid components were separated, collected, and dried with baking to give aluminum 12-hydroxyoctadecanoate (Al³⁺ (⁻OOC-C₁₇-OH)₃). (3) 10 mmol Al³⁺ (⁻OOC-C₁₇-OH)₃ and 300 mmol PDO were mixed in a round-bottom flask. The mixture was heated and reacted at 120 °C in an oil bath under nitrogen for 4 h. Then, the mixture was cooled to 60 °C and reacted for 16 h to give Al³⁺ [⁻OOC-C₁₇-poly(PDO)₁₀-OH]₃ through ring-opening polymerization. The long-chain structure of the product of step (3) was a polyether-ester chain, and the number of carbon atoms in the main chain was 4*10+18 = 58.

### Example 48

(1) 30 mmol 8-hydroxyoctanoic acid and 30 mmol sodium hydroxide were mixed in 50 mL of water, and the mixture was stirred at 90 °C for 0.5 h to give an aqueous solution of sodium 8-hydroxyoctanoate. (2) Then, 10 mmol AlCl₃ was added, and the mixture was stirred at 90 °C for another 0.5 h. Then, the mixture was cooled to room temperature, and the solid components were separated, collected, and dried with baking to give aluminum 8-hydroxyoctanoate (Al³⁺ (⁻OOC-C₇-OH)₃). (3) 10 mmol Al³⁺ (⁻OOC-C₇-OH)₃ and 300 mmol PDO were mixed in a round-bottom flask. The mixture was heated and reacted at 120 °C in an oil bath under nitrogen for 4 h. Then, the mixture was cooled to 60 °C and reacted for 16 h to give Al³⁺ [⁻OOC-C₇-poly(PDO)₁₀-OH]₃ through ring-opening polymerization. The long-chain structure of the product of step (3) was a polyether-ester chain, and the number of carbon atoms in the main chain was 4*10+8=48.

### Example 49

(1) 30 mmol 2-hydroxyethanesulfonic acid and 30 mmol sodium hydroxide were mixed in 50 mL of water, and the mixture was stirred at 90 °C for 0.5 h to give an aqueous solution of sodium 2-hydroxyethanesulfonate. (2) Then, 10 mmol AlCl₃ was added, and the mixture was stirred for another 2 h. Then, the mixture was rotary evaporated to remove the water in the system, and dried with baking to give aluminum 2-hydroxyethanesulfonate (Al³⁺ (⁻ SO₃-C₂-OH)₃). (3) 10 mmol Al³⁺ (⁻SO₃-C₂-OH)₃ and 300 mmol PDO were mixed in a round-bottom flask. The mixture was heated and reacted at 120 °C in an oil bath under nitrogen for 4 h. Then, the mixture was cooled to 60 °C and reacted for 16 h to give Al³⁺ [⁻ SO₃-C₂-poly(PDO)₁₀-OH]₃ through ring-opening polymerization. The long-chain structure of the product of step (3) was a polyether-ester chain, and the number of carbon atoms in the main chain was 4*10+2=42.

### Example 50

Step (1) and step (2) were the same as example 31 (1) and (2). (3) 10 mmol Al³⁺ [⁻ OOC-C₁₁(C₆)-OH]₃ and 300 mmol BDXOP were mixed in a round-bottom flask. The mixture was heated and reacted at 140 °C in an oil bath under nitrogen for 4 h. Then, the mixture was cooled to 80 °C and reacted for 12 h to give Al³⁺ [⁻OOC-C₁₁(C₆)-poly(BDXOP)₁₀-OH]₃ through ring-opening polymerization. The long-chain structure of the product of step (3) was a polyether-ester chain, and the number of carbon atoms in the main chain was 5*10+12=62.

### Example 51

Step (1) and step (2) were the same as example 31 (1) and (2). (3) 10 mmol Al³⁺ [⁻ OOC-C₁₁(C₆)-OH]₃ and 30 mmol δ-VL were mixed in a round-bottom flask. The mixture was heated and reacted at 140 °C in an oil bath under nitrogen for 1 h to give Al³⁺ [⁻ OOC-C₁₁(C₆)-poly(δ-VL)₁-OH]₃ through ring-opening polymerization. The long-chain structure of the product of step (3) was an aliphatic polyester chain, and the number of carbon atoms in the main chain was 5*1+12=17.

### Example 52

Step (1) and step (2) were the same as example 31 (1) and (2). (3) 10 mmol Al³⁺ [⁻ OOC-C₁₁(C₆)-OH]₃ and 3000 mmol δ-VL were mixed in a round-bottom flask. The mixture was heated and reacted at 140 °C in an oil bath under nitrogen for 48 h to give Al³⁺ [⁻ OOC-C₁₁(C₆)-poly(δ-VL)₁₀₀-OH]₃ through ring-opening polymerization. The long-chain structure of the product of step (3) was an aliphatic polyester chain, and the number of carbon atoms in the main chain was 5*100+12=512.

### Example 53

Step (1) and step (2) were the same as example 31 (1) and (2). (3) 10 mmol Al³⁺ [⁻ OOC-C₁₁(C₆)-OH]₃ and 600 mmol L-lactic acid were added into a round-bottom flask. The mixture was reacted successively at a controlled temperature of 80, 100, 120, and 140 °C in an oil bath for 2 h, respectively, at a pressure of 3000 Pa reduced by a water pump. Then, the mixture was reacted at 140 °C and a pressure of 100 Pa reduced by an oil pump for 8 h to give Al³⁺ [⁻OOC-C₁₁(C₆)-poly(L-LA)₁₀-OH]₃ through polycondensation. The long-chain structure of the product of step (3) was an aliphatic polyester chain, and the number of carbon atoms in the main chain was 4*10+12=52.

### Example 54

Step (1) and step (2) were the same as example 31 (1) and (2). (3) 10 mmol Al³⁺ [⁻ OOC-C₁₁(C₆)-OH]₃ and 1500 mmol glycolic acid were added into a round-bottom flask. The mixture was reacted successively at a controlled temperature of 80, 100, 120, and 140 °C in an oil bath for 2 h, respectively, at a pressure of 3000 Pa reduced by a water pump. Then, the mixture was reacted at 160 °C at a pressure of 100 Pa reduced by an oil pump for 6 h to give Al³⁺ [⁻OOC-C₁₁(C₆)-poly(GA)₂₅-OH]₃ through polycondensation. The long-chain structure of the product of step (3) was an aliphatic polyester chain, and the number of carbon atoms in the main chain was 4*25+12=112.

### Example 55

Step (1) and step (2) were the same as example 31 (1) and (2). (3) 10 mmol Al³⁺ [⁻ OOC-C₁₁(C₆)-OH]₃, 30 mmol succinic acid and 30 mmol ethylene glycol were added into a round-bottom flask. The mixture was reacted successively at a controlled temperature of 80, 100, 120, and 140 °C in an oil bath for 2 h, respectively, at a pressure of 3000 Pa reduced by a water pump to give Al³⁺ [⁻OOC-C₁₁(C₆)-poly(ES)₁-OH]₃ through polycondensation. The long-chain structure of the product of step (3) was an aliphatic polyester chain, and the number of carbon atoms in the main chain was 6*1+12=18.

### Example 56

Step (1) and step (2) were the same as example 31 (1) and (2). (3) 10 mmol Al³⁺ [⁻ OOC-C₁₁(C₆)-OH]₃, 300 mmol succinic acid and 300 mmol butanediol were added into a round-bottom flask. The mixture was reacted successively at a controlled temperature of 80, 100, 120, and 140 °C in an oil bath for 2 h, respectively, at a pressure of 3000 Pa reduced by a water pump. Then, the mixture was reacted at 160 °C and a pressure of 100 Pa reduced by an oil pump for 8 h to give Al³⁺ [⁻OOC-C₁₁(C₆)-poly(BS)₁₀-OH]₃ through polycondensation. The long-chain structure of the product of step (3) was an aliphatic polyester chain, and the number of carbon atoms in the main chain was 8*10+12=92.

### Example 57

Step (1) and step (2) were the same as example 31 (1) and (2). (3) 10 mmol Al³⁺ [⁻ OOC-C₁₁(C₆)-OH]₃, 2100 mmol succinic acid and 2100 mmol hexanediol were added into a round-bottom flask. The mixture was reacted successively at a controlled temperature of 80, 100, 120, and 140 °C in an oil bath for 2 h, respectively, at a pressure of 3000 Pa reduced by a water pump. Then, the mixture was reacted at 160 °C and a pressure of 100 Pa reduced by an oil pump for 40 h to give Al³⁺ [⁻OOC-C₁₁(C₆)-poly(HS)₇₀-OH]₃ through polycondensation. The long-chain structure of the product of step (3) was an aliphatic polyester chain, and the number of carbon atoms in the main chain was 10*70+12=712.

### Example 58

Step (1) and step (2) were the same as example 31 (1) and (2). (3) 10 mmol Al³⁺ [⁻ OOC-C₁₁(C₆)-OH]₃, 300 mmol adipic acid and 300 mmol ethylene glycol were added into a round-bottom flask. The mixture was reacted successively at a controlled temperature of 120, 140, 160, and 180 °C in an oil bath for 2 h, respectively, at a pressure of 3000 Pa reduced by a water pump. Then, the mixture was reacted at 220 °C and a pressure of 100 Pa reduced by an oil pump for 2 h to give Al³⁺ [⁻OOC-C₁₁(C₆)-poly(EA)₁₀-OH]₃ through polycondensation. The long-chain structure of the product of step (3) was an aliphatic polyester chain, and the number of carbon atoms in the main chain was 8*10+12=92.

### Example 59

Step (1) and step (2) were the same as example 31 (1) and (2). (3) 10 mmol Al³⁺ [⁻ OOC-C₁₁(C₆)-OH]₃, 300 mmol adipic acid and 300 mmol butanediol were added into a round-bottom flask. The mixture was reacted successively at a controlled temperature of 80, 100, 120, and 140 °C in an oil bath for 2 h, respectively, at a pressure of 1*10⁴ Pa reduced by a water pump. Then, the mixture was reacted at 160 °C and a pressure of 100 Pa reduced by an oil pump for 12 h to give Al³⁺ [⁻OOC-C₁₁(C₆)-poly(BA)₁₀-OH]₃ through polycondensation. The long-chain structure of the product of step (3) was an aliphatic polyester chain, and the number of carbon atoms in the main chain was 10*10+12=112.

### Example 60

Step (1) and step (2) were the same as example 31 (1) and (2). (3) 10 mmol Al³⁺ [⁻ OOC-C₁₁(C₆)-OH]₃, 3000 mmol adipic acid and 3000 mmol hexanediol were added into a round-bottom flask. The mixture was reacted successively at a controlled temperature of 80, 100, 120, and 140 °C in an oil bath for 2 h, respectively, at a pressure of 3000 Pa reduced by a water pump. Then, the mixture was reacted at 160 °C and a pressure of 1 Pa reduced by an oil pump for 40 h to give Al³⁺ [⁻OOC-C₁₁(C₆)-poly(HA)₁₀₀-OH]₃ through polycondensation. The long-chain structure of the product of step (3) was an aliphatic polyester chain, and the number of carbon atoms in the main chain was 12*100+12=1212.

### Example 61

(1) 100 mmol (based on the number of ester bonds in the polymer) PES and 10 mmol sodium hydroxide were mixed, and the mixture was stirred at 160 °C for 12 h to give poly(ethylene succinate) containing a terminal sodium carboxylate and a terminal hydroxyl (Na^{+ -}OOC-poly(ES)₁₀-OH). (2) 6 mmol Na^{+ -}OOC-poly(ES)₁₀-OH and 3 mmol ZnCl₂ were mixed, and the mixture was stirred at 160 °C for 2 h to give Zn²⁺ [⁻OOC-poly(ES)₁₀-OH]₂. The long-chain structure of the product of step (2) was an aliphatic polyester chain, and the number of carbon atoms in the main chain was 6*10=60.

### Example 62

(1) 100 mmol (based on the number of ester bonds in the polymer) PBS and 100 mmol potassium hydroxide were mixed, and the mixture was stirred at 120 °C for 48 h to give polybutylene succinate containing a terminal potassium carboxylate and a terminal hydroxyl (K^{+ -}OOC-poly(ES)₁-OH). (2) 6 mmol K^{+ -}OOC-poly(BS)₁-OH and 3 mmol ZnCl₂ were mixed, and the mixture was stirred at 160 °C for 2 h to give Zn²⁺ [⁻OOC-poly(BS)₁-OH]₂. The long-chain structure of the product of step (2) was an aliphatic polyester chain, and the number of carbon atoms in the main chain was 8*1=8.

### Example 63

200 mmol (based on the number of ester bonds in the polymer) PHS and 1 mmol magnesium hydroxide were mixed, and the mixture was stirred at 180 °C for 2 h to give Mg²⁺ [⁻OOC-poly(HS)₁₀₀-OH]₂. The long-chain structure of the product was an aliphatic polyester chain, and the number of carbon atoms in the main chain was 10*100=1000.

### Example 64

100 mmol (based on the number of ester bonds in the polymer) PEA and 1 mmol calcium hydroxide were mixed, and the mixture was stirred at 200 °C for 1 h to give Ca²⁺ [⁻ OOC-poly(EA)₅₀-OH]₂. The long-chain structure of the product was an aliphatic polyester chain, and the number of carbon atoms in the main chain was 8*50=400.

### Example 65

100 mmol (based on the number of ester bonds in the polymer) PBA and 5 mmol tetramethylammonium hydroxide were mixed, and the mixture was stirred at 140 °C for 6 h to give Me₄N^{+ -}OOC-poly(BA)₂₀-OH. The long-chain structure of the product was an aliphatic polyester chain, and the number of carbon atoms in the main chain was 10*20=200.

### Example 66

100 mmol (based on the number of ester bonds in the polymer) PHA and 10 mmol tetraethylammonium hydroxide were mixed, and the mixture was stirred at 120 °C for 8 h to give Et₄N^{+ -}OOC-poly(HA)₁₀-OH. The long-chain structure of the product was an aliphatic polyester chain, and the number of carbon atoms in the main chain was 12*10=120.

### Example 67

(1) 200 mmol (based on the number of ester bonds in the polymer) PLLA and 10 mmol sodium hydroxide were mixed, and the mixture was stirred at 180 °C for 1 h to give poly-L-lactide containing a terminal sodium carboxylate and a terminal hydroxyl (Na^{+ -} OOC-poly(L-LA)₁₀-OH). (2) 6 mmol Na^{+ -}OOC-poly(L-LA)₁₀-OH and 3 mmol ZnCl₂ were mixed, and the mixture was stirred at 180 °C for 1 h to give poly-L-lactide containing a terminal zinc carboxylate and a terminal hydroxyl (Zn²⁺ [⁻OOC-poly(L-LA)₁₀-OH]₂). The long-chain structure of the product of step (2) was an aliphatic polyester chain, and the number of carbon atoms in the main chain was 4*10=40.

### Example 68

(1) 200 mmol (based on the number of ester bonds in the polymer) PGA and 10 mmol sodium hydroxide were mixed, and the mixture was stirred at 220 °C for 1 h to give polyglycolide containing a terminal sodium carboxylate and a terminal hydroxyl (Na^{+ -} OOC-poly(GA)₁₀-OH). (2) 6 mmol Na^{+ -}OOC-poly(GA)₁₀-OH and 3 mmol ZnCl₂ were mixed, and the mixture was stirred at 220 °C for 0.5 h to give polyglycolide containing a terminal zinc carboxylate and a terminal hydroxyl (Zn²⁺ [⁻OOC-poly(GA)₁₀-OH]₂). The long-chain structure of the product of step (2) was an aliphatic polyester chain, and the number of carbon atoms in the main chain was 4*10=40.

### Example 69

(1) 145 mmol (based on the number of ester bonds in the polymer) PCL and 10 mmol sodium hydroxide were mixed, and the mixture was stirred at 100 °C for 12 h to give polycaprolactone containing a terminal sodium carboxylate and a terminal hydroxyl (Na^{+ -} OOC-poly(ε-CL)_{14.5}-OH). (2) 6 mmol Na^{+ -}OOC-poly(ε-CL)_{14.5}-OH and 3 mmol ZnCl₂ were mixed, and the mixture was stirred at 120 °C for 2 h to give polycaprolactone containing a terminal zinc carboxylate and a terminal hydroxyl (Zn²⁺ [⁻OOC-poly(ε-CL)_{14.5}-OH]₂). The long-chain structure of the product of step (2) was an aliphatic polyester chain, and the number of carbon atoms in the main chain was 6*14.5=87.

### Example 70

(1) 100 mmol (based on the number of ester bonds in the polymer) PVL and 10 mmol sodium hydroxide were mixed, and the mixture was stirred at 60 °C for 48 h to give polyvalerolactone containing a terminal sodium carboxylate and a terminal hydroxyl (Na^{+ -} OOC-poly(δ-VL)₁₀-OH). (2) 6 mmol Na^{+ -}OOC-poly(δ-VL)₁₀-OH and 3 mmol ZnCl₂ were mixed, and the mixture was stirred at 100 °C for 2 h to give polyvalerolactone containing a terminal zinc carboxylate and a terminal hydroxyl (Zn²⁺ [⁻OOC-poly(δ-VL)₁₀-OH]₂). The long-chain structure of the product of step (2) was an aliphatic polyester chain, and the number of carbon atoms in the main chain was 5*10=50.

### Example 71

(1) 100 mmol (based on the number of ester bonds in the polymer) PPDO and 10 mmol sodium hydroxide were mixed, and the mixture was stirred at 120 °C for 14 h to give polydioxanone containing a terminal sodium carboxylate and a terminal hydroxyl (Na^{+ -} OOC-poly(PDO)₁₀-OH). (2) 6 mmol Na^{+ -}OOC-poly(PDO)₁₀-OH and 6.6 mmol ZnCl₂ were mixed, and the mixture was stirred at 120 °C for 2 h to give polydioxanone containing a terminal zinc carboxylate and a terminal hydroxyl (Zn²⁺ [⁻OOC-poly(PDO)₁₀-OH]₂). The long-chain structure of the product of step (2) was a polyether-ester chain, and the number of carbon atoms in the main chain was 4*10=40.

### Example 72

(1) 100 mmol (based on the number of ester bonds in the polymer) PBDXO and 10 mmol sodium hydroxide were mixed, and the mixture was stirred at 160 °C for 8 h to give polyether-ester containing a terminal sodium carboxylate and a terminal hydroxyl (Na^{+ -} OOC-poly(BDXOP)₁₀-OH). (2) 6 mmol Na^{+ -}OOC-poly(BDXO)₁₀-OH and 2.4 mmol ZnCl₂ were mixed, and the mixture was stirred at 140 °C for 2 h to give Zn²⁺ [⁻ OOC-poly(BDXO)₁₀-OH]₂. The long-chain structure of the product of step (2) was a polyether-ester chain, and the number of carbon atoms in the main chain was 5*10=50.

### Example 73

(1) 100 mmol (based on the number of ester bonds in the polymer) PBDXOP and 10 mmol sodium hydroxide were mixed, and the mixture was stirred at 160 °C for 10 h to give polyether-ester containing a terminal sodium carboxylate and a terminal hydroxyl (Na^{+ -} OOC-poly(BDXOP)₁₀-OH). (2) 6 mmol Na^{+ -}OOC-poly(BDXOP)₁₀-OH and 3 mmol ZnCl₂ were mixed, and the mixture was stirred at 140 °C for 2 h to give Zn²⁺ [⁻ OOC-poly(BDXOP)₁₀-OH]₂. The long-chain structure of the product of step (2) was a polyether-ester chain, and the number of carbon atoms in the main chain was 5*10=50.

### Example 74

(1) 10 mmol (based on the terminal carboxylic acid) polyethylene glycol containing a terminal carboxylic acid and a terminal hydroxyl (HOOC-poly(EG)₁₀₀-OH) and 10 mmol sodium hydroxide were mixed, and the mixture was stirred at 60 °C for 4 h to give polyethylene glycol containing a terminal sodium carboxylate and a terminal hydroxyl (Na^{+ -} OOC-poly(EG)₁₀₀-OH). (2) 6 mmol Na^{+ -}OOC-poly(EG)₁₀₀-OH and 3 mmol ZnCl₂ were mixed, and the mixture was stirred at 25 °C for 4 h to give Zn²⁺ [⁻OOC-poly(EG)₁₀₀-OH]₂. The long-chain structure of the product of step (2) was a polyether chain, and the number of carbon atoms in the main chain was 2*100=200.

### Example 75

(1) 10 mmol (based on the terminal carboxylic acid) polytetrahydrofuran containing a terminal carboxylic acid and a terminal hydroxyl (HOOC-poly(THF)₅₀-OH) and 8 mmol sodium hydroxide were mixed, and the mixture was stirred at 120 °C for 0.5 h to give polytetrahydrofuran containing a terminal sodium carboxylate and a terminal hydroxyl (Na^{+ -} OOC-poly(THF)₅₀-OH). (2) Then, 4 mmol ZnCl₂ was added and mixed, and the mixture was stirred at 160 °C for 2 h to give Zn²⁺ (⁻OOC-poly(THF)₅₀-OH)₂. The long-chain structure of the product of step (2) was a polyether chain, and the number of carbon atoms in the main chain was 4*50=200.

### Example 76

(1) 10 mmol (based on the terminal carboxylic acid) HOOC-poly(EG)₁₀₀-OH and 12 mmol sodium hydroxide were mixed, and the mixture was stirred at 60 °C for 2 h to give Na^{+ -} OOC-poly(EG)₁₀₀-OH. (2) Then, 6 mmol ZnCl₂ was added and mixed, and the mixture was stirred at 25 °C for 12 h to give Zn²⁺ [⁻OOC-poly(EG)₁₀₀-OH]₂. The long-chain structure of the product of step (2) was a polyether chain, and the number of carbon atoms in the main chain was 2*100=200.

### Example 77

(1) 10 mmol (based on the terminal carboxylic acid) HOOC- poly(EG)₁₀₀-OH and 9 mmol sodium hydroxide were mixed, and the mixture was stirred at 80 °C for 1 h to give Na^{+ -} OOC-poly(EG)₁₀₀-OH. (2) Then, 9 mmol SnCl₂ was added and mixed, and the mixture was stirred at 25 °C for 12 h to give Sn²⁺ [⁻OOC-poly(EG)₁₀₀-OH]₂. The long-chain structure of the product of step (2) was a polyether chain, and the number of carbon atoms in the main chain was 2*100=200.

### Example 78

(1) 30 mmol (based on the terminal carboxylic acid) HOOC- poly(EG)₁₀₀-OH and 33 mmol sodium hydroxide were mixed, and the mixture was stirred at 25 °C for 12 h to give Na⁺ OOC-poly(EG)₁₀₀-OH. (2) Then, 11 mmol FeCl₃ was added and mixed, and the mixture was stirred at 25 °C for 12 h to give Fe³⁺ [⁻OOC-poly(EG)₁₀₀-OH]₃. The long-chain structure of the product of step (2) was a polyether chain, and the number of carbon atoms in the main chain was 2*100=200.

### Example 79

(1) 30 mmol (based on the terminal carboxylic acid) HOOC- poly(EG)₁₀₀-OH and 30 mmol sodium hydroxide were mixed, and the mixture was stirred at 40 °C for 4 h to give Na^{+ -} OOC-poly(EG)₁₀₀-OH. (2) Then, 10 mmol AlCl₃ was added and mixed, and the mixture was stirred at 25 °C for 12 h to give Al³⁺ [⁻OOC-poly(EG)₁₀₀-OH]₃. The long-chain structure of the product of step (2) was a polyether chain, and the number of carbon atoms in the main chain was 2*100=200.

### Example 80

(1) 10 mmol (based on the terminal carboxylic acid) HOOC- poly(EG)₁₀₀-OH and 10 mmol sodium hydroxide were mixed, and the mixture was stirred at 40 °C for 8 h to give Na^{+ -} OOC-poly(EG)₁₀₀-OH. (2) Then, 7.5 mmol ZnCl₂ was added and mixed, and the mixture was stirred at 25 °C for 12 h to give Zn²⁺ [⁻OOC-poly(EG)₁₀₀-OH]₂. The long-chain structure of the product of step (2) was a polyether chain, and the number of carbon atoms in the main chain was 2*100=200.

### Application example 1

The Cl^{- +}MIm-C₈-OH prepared in step (1) of example 1 was used to catalyze the hydrolysis of PVL. The molar ratio of the terminal hydroxyl of the catalyst to the ester bond of PVL was 1/100, and the mass ratio of the added water to PVL was 1/1. The mixture was hydrolyzed at 140 °C for 4 h in a hydrothermal reactor, and then cooled to room temperature. The depolymerization conversion rate was 98%.

### Application example 2

The Cl^{- +}MIm-C₈-OH prepared in step (1) of example 1 was used to catalyze the alcoholysis of PVL. The molar ratio of the terminal hydroxyl of the catalyst to the ester bond of PVL was 1/100, and the mass ratio of the added methanol to PVL was 2/1. The mixture was alcoholyzed at 140 °C for 4 h in a hydrothermal reactor, and then cooled to room temperature. The depolymerization conversion rate was 99%.

### Application example 3

The Cl^{- +}MIm-C₈-OH prepared in step (1) of example 1 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 56%.

### Application example 4

The FeCl₃-Cl^{- +}MIm-C₈-OH prepared in step (2) of example 1 was used to catalyze the hydrolysis of PVL. The molar ratio of the terminal hydroxyl of the catalyst to the ester bond of PVL was 1/100, and the mass ratio of the added water to PVL was 1.5/1. The mixture was hydrolyzed at 140 °C for 3 h in a hydrothermal reactor, and then cooled to room temperature. The depolymerization conversion rate was 99%.

### Application example 5

The FeCl₃-Cl^{- +}MIm-C₈-OH prepared in step (2) of example 1 was used to catalyze the alcoholysis of PVL. The molar ratio of the terminal hydroxyl of the catalyst to the ester bond of PVL was 1/100, and the mass ratio of the added methanol to PVL was 2/1. The mixture was alcoholyzed at 140 °C for 3 h in a hydrothermal reactor, and then cooled to room temperature. The depolymerization conversion rate was 99%.

### Application example 6

The FeCl₃-Cl^{- +}MIm-C₈-OH prepared in step (2) of example 1 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 85%.

### Application example 7

The FeCl₃-Cl^{- +}MIm-C₈-poly(δ-VL)₁₀-OH prepared in step (3) of example 1 was used to catalyze the hydrolysis of PVL. The molar ratio of the terminal hydroxyl of the catalyst to the ester bond of PVL was 1/100, and the mass ratio of the added water to PVL was 1.2/1. The mixture was hydrolyzed at 140 °C for 2 h in a hydrothermal reactor, and then cooled to room temperature. The depolymerization conversion rate was 100%.

### Application example 8

The FeCl₃-Cl^{- +}MIm-C₈-poly(δ-VL)₁₀-OH prepared in step (3) of example 1 was used to catalyze the alcoholysis of PVL. The molar ratio of the terminal hydroxyl of the catalyst to the ester bond of PVL was 1/100, and the mass ratio of the added methanol to PVL was 5/1. The mixture was alcoholyzed at 140 °C for 2 h in a hydrothermal reactor, and then cooled to room temperature. The depolymerization conversion rate was 100%.

### Application example 9

The FeCl₃-Cl^{- +}MIm-C₈-poly(δ-VL)₁₀-OH prepared in step (3) of example 1 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1.5 h, the yield of depolymerization was 98%. After 2 h, the yield of depolymerization was 107%. (due to the presence of -poly(δ-VL)- segments in the catalyst, the catalyst would depolymerize when over-reacted, resulting in the mass of the product being greater than the mass of the reactant PVL).

### Application example 10

The FeCl₃-Cl^{- +}MIm-C₃-poly(δ-VL)₅-OH prepared in step (3) of example 2 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1.5 h, the yield of depolymerization was 97%.

### Application example 11

The FeCl₃-Br^{- +}MIm-C₂-poly(δ-VL)₃₀-OH prepared in step (3) of example 3 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1.5 h, the yield of depolymerization was 94%.

### Application example 12

The FeCl₃-Cl^{- +}BIm-C₃-poly(δ-VL)₅₀-OH prepared in step (3) of example 4 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1.5 h, the yield of depolymerization was 107%.

### Application example 13

The FeCl₃-Cl^{- +}MThi-C₃-poly(δ-VL)₇₀-OH prepared in step (3) of example 5 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1.5 h, the yield of depolymerization was 109%.

### Application example 14

The FeCl₃-Cl^{- +}Thi-C₃-poly(δ-VL)₉₀-OH prepared in step (3) of example 6 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1.5 h, the yield of depolymerization was 118%.

### Application example 15

The FeCl₃-Cl^{- +}BzThi-C₃-poly(δ-VL)₁₀-OH prepared in step (3) of example 7 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1.5 h, the yield of depolymerization was 96%.

### Application example 16

The SnCl₂-Cl^{- +}MIm-C₃-poly(δ-VL)₁₀-OH prepared in step (3) of example 8 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 98%.

### Application example 17

The ZnCl₂-Cl^{- +}MIm-C₈-poly(δ-VL)₁₀-OH prepared in step (3) of example 9 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 94%.

### Application example 18

The AlCl₃-Cl^{- +}MIm-C₃-poly(δ-VL)₁₀-OH prepared in step (3) of example 10 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 79%.

### Application example 19

The 1.7·FeCl₃-Cl^{- +}MIm-C₃-poly(δ-VL)₁₀-OH prepared in step (3) of example 11 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 92%.

### Application example 20

The 3·FeCl₃-Cl^{- +}MIm-C₃-poly(δ-VL)₁₀-OH prepared in step (3) of example 12 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 43%, and the reaction system was obviously carbonized.

### Application example 21

The 1.3·FeCl₃-Cl^{- +}MIm-C₃-poly(δ-VL)₁₀-OH prepared in step (3) of example 13 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 90%.

### Application example 22

The FeCl₃-Cl^{- +}MIm-C₃-poly(L-LA)₁₀-OH prepared in step (3) of example 14 was used to catalyze the cyclodepolymerization of PLLA. The molar ratio of the terminal hydroxyl of the catalyst to the ester bond of PLLA was 1/50. The mixture was stirred at 200 °C and a pressure of 100 Pa reduced by an oil pump, and a product of cyclodepolymerization was continuously distilled out. After 0.75 h, the yield of depolymerization was 96%. After 1 h, the yield of depolymerization was 104%. The product was subjected to ¹H NMR spectroscopy, and the results were shown in Fig. 7, which showed that the recovered product was high-purity L-LA; the product was subjected to gas chromatography, and the results were shown in Fig. 8, which showed that the content of L-LA in the recovered product was greater than 98%, and the content of meso-LA was about 1%.

### Application example 23

The FeCl₃-Cl^{- +}MIm-C₃-poly(GA)₁₀-OH prepared in step (3) of example 15 was used to catalyze the cyclodepolymerization of PGA. The molar ratio of the terminal hydroxyl of the catalyst to the ester bond of PGA was 1/100. The mixture was stirred at 220 °C and a pressure of 100 Pa reduced by an oil pump, and a product of cyclodepolymerization was continuously distilled out. After 1 h, the yield of depolymerization was 98%.

### Application example 24

The FeCl₃-Cl^{- +}MIm-C₃-poly(ε-CL)₁₀-OH prepared in step (3) of example 16 was used to catalyze the cyclodepolymerization of PCL. The molar ratio of the terminal hydroxyl of the catalyst to the ester bond of PCL was 1/100. The mixture was stirred at 200 °C and a pressure of 100 Pa reduced by an oil pump, and a product of cyclodepolymerization was continuously distilled out. After 4 h, the yield of depolymerization was 98%. After 6 h, the yield of depolymerization was 107%. The product was subjected to ¹H NMR spectroscopy, and the results were shown in Fig. 9, which showed that the recovered product was a mixture of -CL monomers, cyclic dimers and cyclic trimers; the product was subjected to gas chromatography, and the results were shown in Fig. 10, which showed that the proportions of ε-CL monomers, cyclic dimers and cyclic trimers in the recovered product were 82.90%, 15.23% and 1.76%, respectively.

### Application example 25

The FeCl₃-Cl^{- +}MIm-C₃-poly(PDO)₁₀-OH prepared in step (3) of example 17 was used to catalyze the cyclodepolymerization of PPDO. The molar ratio of the terminal hydroxyl of the catalyst to the ester bond of PPDO was 1/100. The mixture was stirred at 160 °C and a pressure of 100 Pa reduced by an oil pump, and a product of cyclodepolymerization was continuously distilled out. After 1 h, the yield of depolymerization was 99%. The product was subjected to ¹H NMR spectroscopy, and the results were shown in Fig. 11, which showed that the recovered product was high purity PDO; the product was subjected to gas chromatography, and the results were shown in Fig. 12, which showed that the content of PDO monomers in the recovered product was 99.61%, and the content of PDO cyclic dimers was 0.28%.

### Application example 26

The FeCl₃-Cl^{- +}MIm-C₃-poly(PDO)₁₀-OH prepared in step (3) of example 18 was used to catalyze the cyclodepolymerization of PPDO. The molar ratio of the terminal hydroxyl of the catalyst to the ester bond of PPDO was 1/100. The mixture was stirred at 140 °C and a pressure of 100 Pa reduced by an oil pump, and a product of cyclodepolymerization was continuously distilled out. After 1 h, the yield of depolymerization was 97%.

### Application example 27

The FeCl₃-Cl^{- +}MIm-C₃-poly(BDXO)₁₀-OH prepared in step (3) of example 19 was used to catalyze the cyclodepolymerization of PBDXO. The molar ratio of the terminal hydroxyl of the catalyst to the ester bond of PBDXO was 1/100. The mixture was stirred at 160 °C and a pressure of 100 Pa reduced by an oil pump, and a product of cyclodepolymerization was continuously distilled out. After 2 h, the yield of depolymerization was 94%.

### Application example 28

The FeCl₃-Cl^{- +}MIm-C₃-poly(BDXOP)₁₀-OH prepared in step (3) of example 20 was used to catalyze the cyclodepolymerization of PBDXOP. The molar ratio of the terminal hydroxyl of the catalyst to the ester bond of PBDXOP was 1/100. The mixture was stirred at 160 °C and a pressure of 100 Pa reduced by an oil pump, and a product of cyclodepolymerization was continuously distilled out. After 2 h, the yield of depolymerization was 90%.

### Application example 29

The FeCl₃-Cl^{- +}MIm-C₃-poly(δ-VL)₁-OH prepared in step (3) of example 21 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1.5 h, the yield of depolymerization was 56%.

### Application example 30

The FeCl₃-Cl^{- +}MIm-C₃-poly(δ-VL)₁₀₀-OH prepared in step (3) of example 22 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1.5 h, the yield of depolymerization was 80%.

### Application example 31

The FeCl₃-Cl^{- +}MIm-C₃-poly(L-LA)₁₀-OH prepared in step (3) of example 23 was used to catalyze the cyclodepolymerization of PLLA. The molar ratio of the terminal hydroxyl of the catalyst to the ester bond of PLLA was 1/100. The mixture was stirred at 200 °C and a pressure of 100 Pa reduced by an oil pump, and a product of cyclodepolymerization was continuously distilled out. After 0.75 h, the yield of depolymerization was 98%.

### Application example 32

The FeCl₃-Cl^{- +}MIm-C₃-poly(GA)₂₅-OH prepared in step (3) of example 24 was used to catalyze the cyclodepolymerization of PGA. The molar ratio of the terminal hydroxyl of the catalyst to the ester bond of PGA was 1/100. The mixture was stirred at 220 °C and a pressure of 100 Pa reduced by an oil pump, and a product of cyclodepolymerization was continuously distilled out. After 1 h, the yield of depolymerization was 108%.

### Application example 33

The FeCl₃-Cl^{- +}MIm-C₃-poly(ES)₁-OH prepared in step (3) of example 25 was used to catalyze the hydrolysis of PES. The molar ratio of the terminal hydroxyl of the catalyst to the ester bond of PES was 1/100, and the mass ratio of the added water to PES was 4/1. The mixture was hydrolyzed at 140 °C for 4 h in a hydrothermal reactor, and then cooled to room temperature. The depolymerization conversion rate was 99%.

### Application example 34

The FeCl₃-Cl^{- +}MIm-C₃-poly(BS)₁₀-OH prepared in step (3) of example 26 was used to catalyze the hydrolysis of PBS. The molar ratio of the terminal hydroxyl of the catalyst to the ester bond of PBS was 1/100, and the mass ratio of the added water to PBS was 8/1. The mixture was hydrolyzed at 140 °C for 4 h in a hydrothermal reactor, and then cooled to room temperature. The depolymerization conversion rate was 100%.

### Application example 35

The FeCl₃-Cl^{- +}MIm-C₃-poly(HS)₇₀-OH prepared in step (3) of example 27 was used to catalyze the hydrolysis of PHS. The molar ratio of the terminal hydroxyl of the catalyst to the ester bond of PHS was 1/100, and the mass ratio of the added water to PHS was 2/1. The mixture was hydrolyzed at 140 °C for 4 h in a hydrothermal reactor, and then cooled to room temperature. The depolymerization conversion rate was 97%.

### Application example 36

The FeCl₃-Cl^{- +}MIm-C₃-poly(EA)₁₀-OH prepared in step (3) of example 28 was used to catalyze the hydrolysis of PEA. The molar ratio of the terminal hydroxyl of the catalyst to the ester bond of PEA was 1/100, and the mass ratio of the added water to PEA was 15/1. The mixture was hydrolyzed at 140 °C for 4 h in a hydrothermal reactor, and then cooled to room temperature. The depolymerization conversion rate was 100%.

### Application example 37

The FeCl₃-Cl^{- +}MIm-C₃-poly(BA)₁₀-OH prepared in step (3) of example 29 was used to catalyze the hydrolysis of PBA. The molar ratio of the terminal hydroxyl of the catalyst to the ester bond of PBA was 1/100, and the mass ratio of the added water to PBA was 20/1. The mixture was hydrolyzed at 140 °C for 4 h in a hydrothermal reactor, and then cooled to room temperature. The depolymerization conversion rate was 100%.

### Application example 38

The FeCl₃-Cl^{- +}MIm-C₃-poly(HA)₁₀₀-OH prepared in step (3) of example 30 was used to catalyze the hydrolysis of PHA. The molar ratio of the terminal hydroxyl of the catalyst to the ester bond of PHA was 1/100, and the mass ratio of the added water to PHA was 12/1. The mixture was hydrolyzed at 140 °C for 4 h in a hydrothermal reactor, and then cooled to room temperature. The depolymerization conversion rate was 99%.

### Application example 39

The Al³⁺ [⁻OOC-C₁₁(C₆)-OH]₃ prepared in step (2) of example 31 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 85%.

### Application example 40

The Al³⁺ [⁻OOC-C₁₁(C₆)-poly(δ-VL)₁₀-OH]₃ prepared in step (3) of example 31 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 94%.

### Application example 41

The Al³⁺ [⁻OOC-C₅-poly(δ-VL)₅-OH]₃ prepared in step (3) of example 32 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 95%.

### Application example 42

The Me₄N^{+ -}SO₃-C₄-poly(δ-VL)₃₀-OH prepared in step (2) of example 33 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 59%.

### Application example 43

The Et₄N^{+ -}SO₃-C₃-poly(δ-VL)₅₀-OH prepared in step (2) of example 34 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 47%.

### Application example 44

The Al³⁺ [⁻OOC-C₁₄-poly(δ-VL)₇₀-OH]₃ prepared in step (3) of example 35 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 133%.

### Application example 45

The Al³⁺ [⁻OOC-C₅-poly(δ-VL)₉₀-OH]₃ prepared in step (3) of example 36 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 142%.

### Application example 46

The Mg²⁺ [⁻OOC-C₁₄-poly(δ-VL)₁₀-OH]₂ prepared in step (2) of example 37 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 105%.

### Application example 47

The Ca²⁺ [⁻OOC-C₁₄-poly(δ-VL)₁₀-OH]₂ prepared in step (2) of example 38 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 98%.

### Application example 48

The Fe³⁺ [⁻OOC-C₁₄-poly(δ-VL)₁₀-OH]₃ prepared in step (3) of example 39 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 96%.

### Application example 49

The Zn²⁺ [⁻OOC-C₁₄-poly(δ-VL)₁₀-OH]₂ prepared in step (3) of example 40 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 100%.

### Application example 50

The Sn²⁺ [⁻OOC-C₁₄-poly(δ-VL)₁₀-OH]₂ prepared in step (3) of example 41 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 103%.

### Application example 51

The Al³⁺ [⁻OOC-C₁₄-poly(δ-VL)₁₀-OH]₃ prepared in step (3) of example 42 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 94%.

### Application example 52

The Al³⁺ [⁻OOC-C₁₄-poly(δ-VL)₁₀-OH]₃ prepared in step (3) of example 43 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 94%.

### Application example 53

The Al³⁺ [⁻OOC-C₁₁(C₆)-poly(L-LA)₁₀-OH]₃ prepared in step (3) of example 44 was used to catalyze the cyclodepolymerization of PLLA. The molar ratio of the terminal hydroxyl of the catalyst to the ester bond of PLLA was 1/100. The mixture was stirred at 200 °C and a pressure of 100 Pa reduced by an oil pump, and a product of cyclodepolymerization was continuously distilled out. After 2 h, the yield of depolymerization was 98%.

### Application example 54

The Al³⁺ [⁻OOC-C₁₁(C₆)-poly(GA)₁₀-OH]₃ prepared in step (3) of example 45 was used to catalyze the cyclodepolymerization of PGA. The molar ratio of the terminal hydroxyl of the catalyst to the ester bond of PGA was 1/100. The mixture was stirred at 220 °C and a pressure of 100 Pa reduced by an oil pump, and a product of cyclodepolymerization was continuously distilled out. After 1 h, the yield of depolymerization was 88%.

### Application example 55

The Al³⁺ [⁻OOC-C₁₁(C₆)-poly(ε-CL)₁₀-OH]₃ prepared in step (3) of example 46 was used to catalyze the cyclodepolymerization of PCL. The molar ratio of the terminal hydroxyl of the catalyst to the ester bond of PCL was 1/100. The mixture was stirred at 200 °C and a pressure of 100 Pa reduced by an oil pump, and a product of cyclodepolymerization was continuously distilled out. After 6 h, the yield of depolymerization was 84%.

### Application example 56

The Al³⁺ [⁻OOC-C₁₇-poly(PDO)₁₀-OH]₃ prepared in step (3) of example 47 was used to catalyze the cyclodepolymerization of PPDO. The molar ratio of the terminal hydroxyl of the catalyst to the ester bond of PPDO was 1/100. The mixture was stirred at 160 °C and a pressure of 100 Pa reduced by an oil pump, and a product of cyclodepolymerization was continuously distilled out. After 2 h, the yield of depolymerization was 101%.

### Application example 57

The Al³⁺ [⁻OOC-C₇-poly(PDO)₁₀-OH]₃ prepared in step (3) of example 48 was used to catalyze the cyclodepolymerization of PPDO. The molar ratio of the terminal hydroxyl of the catalyst to the ester bond of PPDO was 1/100. The mixture was stirred at 160 °C and a pressure of 100 Pa reduced by an oil pump, and a product of cyclodepolymerization was continuously distilled out. After 2 h, the yield of depolymerization was 102%.

### Application example 58

The Al³⁺ [⁻SO₃-C₂-poly(PDO)₁₀-OH]₃ prepared in step (3) of example 49 was used to catalyze the cyclodepolymerization of PBDXO. The molar ratio of the terminal hydroxyl of the catalyst to the ester bond of PBDXO was 1/100. The mixture was stirred at 160 °C and a pressure of 100 Pa reduced by an oil pump, and a product of cyclodepolymerization was continuously distilled out. After 4 h, the yield of depolymerization was 89%.

### Application example 59

The Al³⁺ [⁻OOC-C₁₁(C₆)-poly(BDXOP)₁₀-OH]₃ prepared in step (3) of example 50 was used to catalyze the cyclodepolymerization of PBDXOP. The molar ratio of the terminal hydroxyl of the catalyst to the ester bond of PBDXOP was 1/100. The mixture was stirred at 160 °C and a pressure of 100 Pa reduced by an oil pump, and a product of cyclodepolymerization was continuously distilled out. After 4 h, the yield of depolymerization was 83%.

### Application example 60

The Al³⁺ [⁻OOC-C₁₁(C₆)-poly(δ-VL)₁-OH]₃ prepared in step (3) of example 51 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 68%.

### Application example 61

The Al³⁺ [⁻OOC-C₁₁(C₆)-poly(δ-VL)₁₀₀-OH]₃ prepared in step (3) of example 52 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 83%.

### Application example 62

The Al³⁺ [⁻OOC-C₁₁(C₆)-poly(L-LA)₁₀-OH]₃ prepared in step (3) of example 53 was used to catalyze the cyclodepolymerization of PLLA. The molar ratio of the terminal hydroxyl of the catalyst to the ester bond of PLLA was 1/100. The mixture was stirred at 200 °C and a pressure of 100 Pa reduced by an oil pump, and a product of cyclodepolymerization was continuously distilled out. After 2 h, the yield of depolymerization was 100%.

### Application example 63

The Al³⁺ [⁻OOC-C₁₁(C₆)-poly(GA)₂₅-OH]₃ prepared in step (3) of example 54 was used to catalyze the cyclodepolymerization of PGA. The molar ratio of the terminal hydroxyl of the catalyst to the ester bond of PGA was 1/100. The mixture was stirred at 220 °C and a pressure of 100 Pa reduced by an oil pump, and a product of cyclodepolymerization was continuously distilled out. After 1 h, the yield of depolymerization was 101%.

### Application example 64

The Al³⁺ [⁻OOC-C₁₁(C₆)-poly(ES)₁-OH]₃ prepared in step (3) of example 55 was used to catalyze the hydrolysis of PES. The molar ratio of the terminal hydroxyl of the catalyst to the ester bond of PES was 1/100, and the mass ratio of the added water to PES was 2/1. The mixture was hydrolyzed at 140 °C for 4 h in a hydrothermal reactor, and then cooled to room temperature. The depolymerization conversion rate was 99%.

### Application example 65

The Al³⁺ [⁻OOC-C₁₁(C₆)-poly(BS)₁₀-OH]₃ prepared in step (3) of example 56 was used to catalyze the hydrolysis of PBS. The molar ratio of the terminal hydroxyl of the catalyst to the ester bond of PBS was 1/100, and the mass ratio of the added water to PBS was 2/1. The mixture was hydrolyzed at 140 °C for 4 h in a hydrothermal reactor, and then cooled to room temperature. The depolymerization conversion rate was 100%.

### Application example 66

The Al³⁺ [⁻OOC-C₁₁(C₆)-poly(HS)₇₀-OH]₃ prepared in step (3) of example 57 was used to catalyze the hydrolysis of PHS. The molar ratio of the terminal hydroxyl of the catalyst to the ester bond of PHS was 1/10000, and the mass ratio of the added water to PHS was 2/1. The mixture was hydrolyzed at 140 °C for 12 h in a hydrothermal reactor, and then cooled to room temperature. The depolymerization conversion rate was 34%.

### Application example 67

The Al³⁺ [⁻OOC-C₁₁(C₆)-poly(EA)₁₀-OH]₃ prepared in step (3) of example 58 was used to catalyze the hydrolysis of PEA. The molar ratio of the terminal hydroxyl of the catalyst to the ester bond of PEA was 1/1000, and the mass ratio of the added water to PEA was 2/1. The mixture was hydrolyzed at 140 °C for 8 h in a hydrothermal reactor, and then cooled to room temperature. The depolymerization conversion rate was 67%.

### Application example 68

The Al³⁺ [⁻OOC-C₁₁(C₆)-poly(BA)₁₀-OH]₃ prepared in step (3) of example 59 was used to catalyze the hydrolysis of PBA. The molar ratio of the terminal hydroxyl of the catalyst to the ester bond of PBA was 1/200, and the mass ratio of the added water to PBA was 2/1. The mixture was hydrolyzed at 140 °C for 6 h in a hydrothermal reactor, and then cooled to room temperature. The depolymerization conversion rate was 95%.

### Application example 69

The Al³⁺ [⁻OOC-C₁₁(C₆)-poly(HA)₁₀₀-OH]₃ prepared in step (3) of example 60 was used to catalyze the hydrolysis of PHA. The molar ratio of the terminal hydroxyl of the catalyst to the ester bond of PHA was 1/100, and the mass ratio of the added water to PHA was 2/1. The mixture was hydrolyzed at 140 °C for 4 h in a hydrothermal reactor, and then cooled to room temperature. The depolymerization conversion rate was 98%.

### Application example 70

The Zn²⁺ [⁻OOC-poly(ES)₁₀-OH]₂ prepared in step (2) of example 61 was used to catalyze the hydrolysis of PES. The molar ratio of the terminal hydroxyl of the catalyst to the ester bond of PES was 1/100, and the mass ratio of the added water to PES was 2/1. The mixture was hydrolyzed at 140 °C for 3 h in a hydrothermal reactor, and then cooled to room temperature. The depolymerization conversion rate was 100%.

### Application example 71

The Zn²⁺ [⁻OOC-poly(BS)₁-OH]₂ prepared in step (2) of example 62 was used to catalyze the hydrolysis of PBS. The molar ratio of the terminal hydroxyl of the catalyst to the ester bond of PBS was 1/50, and the mass ratio of the added water to PBS was 2/1. The mixture was hydrolyzed at 140 °C for 3 h in a hydrothermal reactor, and then cooled to room temperature. The depolymerization conversion rate was 99%.

### Application example 72

The Mg²⁺ [⁻OOC-poly(HS)₁₀₀-OH]₂ prepared in example 63 was used to catalyze the hydrolysis of PHS. The molar ratio of the terminal hydroxyl of the catalyst to the ester bond of PHS was 1/20, and the mass ratio of the added water to PHS was 2/1. The mixture was hydrolyzed at 140 °C for 3 h in a hydrothermal reactor, and then cooled to room temperature. The depolymerization conversion rate was 100%.

### Application example 73

The Ca²⁺ [⁻OOC-poly(EA)₅₀-OH]₂ prepared in example 64 was used to catalyze the hydrolysis of PEA. The molar ratio of the terminal hydroxyl of the catalyst to the ester bond of PEA was 1/10, and the mass ratio of the added water to PEA was 2/1. The mixture was hydrolyzed at 140 °C for 3 h in a hydrothermal reactor, and then cooled to room temperature. The depolymerization conversion rate was 100%.

### Application example 74

The Me₄N^{+ -}OOC-poly(BA)₂₀-OH prepared in example 65 was used to catalyze the hydrolysis of PBA. The molar ratio of the terminal hydroxyl of the catalyst to the ester bond of PBA was 1/100, and the mass ratio of the added water to PBA was 2/1. The mixture was hydrolyzed at 140 °C for 3 h in a hydrothermal reactor, and then cooled to room temperature. The depolymerization conversion rate was 96%.

### Application example 75

The Et₄N^{+ -}OOC-poly(HA)₁₀-OH prepared in example 66 was used to catalyze the hydrolysis of PHA. The molar ratio of the terminal hydroxyl of the catalyst to the ester bond of PHA was 1/100, and the mass ratio of the added water to PHA was 2/1. The mixture was hydrolyzed at 140 °C for 3 h in a hydrothermal reactor, and then cooled to room temperature. The depolymerization conversion rate was 96%.

### Application example 76

The Zn²⁺ [⁻OOC-poly(L-LA)₁₀-OH]₂ prepared in step (2) of example 67 was used to catalyze the cyclodepolymerization of PLLA. The molar ratio of the terminal hydroxyl of the catalyst to the ester bond of PLLA was 1/100. The mixture was stirred at 200 °C and a pressure of 100 Pa reduced by an oil pump, and a product of cyclodepolymerization was continuously distilled out. After 1 h, the yield of depolymerization was 106%. In the first 0.5 h of the reaction, the viscosity of the reaction system decreased from 570 Pa·s to 22 Pa·s.

### Application example 77

The Zn²⁺ [⁻OOC-poly(GA)₁₀-OH]₂ prepared in step (2) of example 68 was used to catalyze the cyclodepolymerization of PGA. The molar ratio of the terminal hydroxyl of the catalyst to the ester bond of PGA was 1/100. The mixture was stirred at 220 °C and a pressure of 100 Pa reduced by an oil pump, and a product of cyclodepolymerization was continuously distilled out. After 1 h, the yield of depolymerization was 103%.

### Application example 78

The Zn²⁺ [⁻OOC-poly(ε-CL)_{14.5}-OH]₂ prepared in step (2) of example 69 was used to catalyze the cyclodepolymerization of PCL. The molar ratio of the terminal hydroxyl of the catalyst to the ester bond of PCL was 1/100. The mixture was stirred at 200 °C and a pressure of 100 Pa reduced by an oil pump, and a product of cyclodepolymerization was continuously distilled out. After 4 h, the yield of depolymerization was 98%.

### Application example 79

The Zn²⁺ [⁻OOC-poly(δ-VL)₁₀-OH]₂ prepared in step (2) of example 70 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 102%.

### Application example 80

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/100. The mixture was stirred at 160 °C and a pressure of 100 Pa reduced by an oil pump, and a product of cyclodepolymerization was continuously distilled out. After 1 h, the yield of depolymerization was 109%.

### Application example 81

The Zn²⁺ [⁻OOC-poly(BDXO)₁₀-OH]₂ prepared in step (2) of example 72 was used to catalyze the cyclodepolymerization of PBDXO. The molar ratio of the terminal hydroxyl of the catalyst to the ester bond of PBDXO was 1/100. The mixture was stirred at 160 °C and a pressure of 100 Pa reduced by an oil pump, and a product of cyclodepolymerization was continuously distilled out. After 1 h, the yield of depolymerization was 89%.

### Application example 82

The Zn²⁺ [⁻OOC-poly(BDXOP)₁₀-OH]₂ prepared in step (2) of example 73 was used to catalyze the cyclodepolymerization of PBDXOP. The molar ratio of the terminal hydroxyl of the catalyst to the ester bond of PBDXOP was 1/100. The mixture was stirred at 160 °C and a pressure of 100 Pa reduced by an oil pump, and a product of cyclodepolymerization was continuously distilled out. After 1 h, the yield of depolymerization was 94%.

### Application example 83

The Zn²⁺ [⁻OOC-poly(EG)₁₀₀-OH]₂ prepared in step (2) of example 74 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 97%.

### Application example 84

The Zn²⁺ (⁻OOC-poly(THF)₅₀-OH)₂ and HOOC-poly(THF)₅₀-OH prepared in step (2) of example 75 were used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 55%.

### Application example 85

The Zn²⁺ [⁻OOC-poly(EG)₁₀₀-OH]₂ prepared in step (2) of example 76 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 96%.

### Application example 86

The Sn²⁺ [⁻OOC-poly(EG)₁₀₀-OH]₂ prepared in step (2) of example 77 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 105%.

### Application example 87

The Fe³⁺ [⁻OOC-poly(EG)₁₀₀-OH]₃ prepared in step (2) of example 78 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 97%.

### Application example 88

The Al³⁺ [⁻OOC-poly(EG)₁₀₀-OH]₃ prepared in step (2) of example 79 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 2 h, the yield of depolymerization was 92%.

### Application example 89

The Zn²⁺ [⁻OOC-poly(EG)₁₀₀-OH]₂ prepared in step (2) of example 80 was used to catalyze the cyclodepolymerization of PVL. The molar ratio of the terminal hydroxyl of the catalyst to the ester bond of PVL was 1/100. The mixture was stirred at 160 °C and a pressure of 100 Pa reduced by an oil pump, and a product of cyclodepolymerization was continuously distilled out. After 1 h, the yield of depolymerization was 99%.

### Application example 90

The FeCl₃-Cl^{- +}MIm-C₃-poly(BS)₁₀-OH prepared in step (3) of example 26 was used to catalyze the hydrolysis of PBS. The molar ratio of the terminal hydroxyl of the catalyst to the ester bond of PBS was 1/100, and the mass ratio of the added water to PBS was 1/2. The mixture was hydrolyzed at 140 °C for 1 h in a hydrothermal reactor, and then cooled to room temperature. The depolymerization conversion rate was 72%. The product contained succinic acid, butanediol, butylene succinate and oligomers of butylene succinate.

### Application example 91

The FeCl₃-Cl^{- +}MIm-C₃-poly(BS)₁₀-OH prepared in step (3) of example 26 was used to catalyze the hydrolysis of PBS. The molar ratio of the terminal hydroxyl of the catalyst to the ester bond of PBS was 1/100, and the mass ratio of the added water to PBS was 10/1. The mixture was hydrolyzed at 160 °C for 2 h in a hydrothermal reactor, and then cooled to room temperature. The depolymerization conversion rate was 100%.

### Application example 92

The FeCl₃-Cl^{- +}MIm-C₃-poly(BS)₁₀-OH prepared in step (3) of example 26 was used to catalyze the hydrolysis of PBS. The molar ratio of the terminal hydroxyl of the catalyst to the ester bond of PBS was 1/100, and the mass ratio of the added water to PBS was 20/1. The mixture was hydrolyzed at 80 °C for 12 h in a hydrothermal reactor, and then cooled to room temperature. The depolymerization conversion rate was 71%.

### Application example 93

The FeCl₃-Cl^{- +}MIm-C₃-poly(BS)₁₀-OH prepared in step (3) of example 26 was used to catalyze the hydrolysis of PBS. The molar ratio of the terminal hydroxyl of the catalyst to the ester bond of PBS was 1/100, and the mass ratio of the added methanol to PBS was 2/1. The mixture was alcoholyzed at 40 °C for 12 h. The depolymerization conversion rate was 11%.

### Application example 94

The FeCl₃-Cl^{- +}MIm-C₃-poly(BS)₁₀-OH prepared in step (3) of example 26 was used to catalyze the hydrolysis of PBS. The molar ratio of the terminal hydroxyl of the catalyst to the ester bond of PBS was 1/100, and the mass ratio of the added butanediol to PBS was 2/1. The mixture was alcoholyzed at 200 °C for 1 h, and then cooled to room temperature. The depolymerization conversion rate was 100%.

### Application example 95

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/10000. The mixture was stirred at 160 °C and a pressure of 100 Pa reduced by an oil pump, and a product of cyclodepolymerization was continuously distilled out. After 1 h, the yield of depolymerization was 55%.

### Application example 96

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/1000. The mixture was stirred at 160 °C and a pressure of 100 Pa reduced by an oil pump, and a product of cyclodepolymerization was continuously distilled out. After 1 h, the yield of depolymerization was 79%.

### Application example 97

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/10. The mixture was stirred at 120 °C and a pressure of 100 Pa reduced by an oil pump, and a product of cyclodepolymerization was continuously distilled out. After 12 h, the yield of depolymerization was 101%.

### Application example 98

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/100. PPDO and the catalyst were melt blended and then cooled to room temperature, then crushed and passed through a 40-mesh sieve. Then, the mixture was extracted with toluene at 80 °C and atmospheric pressure (1*10⁵ Pa), and monomers were continuously generated and transferred to the extract (toluene). After 12 hours, the depolymerization conversion rate was 47%.

### Application example 99

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/100. The mixture was stirred at 300 °C and a pressure of 100 Pa reduced by an oil pump, and a product of cyclodepolymerization was continuously distilled out. After 0.5 h, the yield of depolymerization was 103%.

### Application example 100

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/100. The mixture was stirred at 400 °C and a pressure of 100 Pa reduced by an oil pump, and a product of cyclodepolymerization was continuously distilled out. After 0.5 h, the yield of depolymerization was 101%.

### Application example 101

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/100. The mixture was stirred at 160 °C and a pressure of 1 Pa reduced by an oil pump, and a product of cyclodepolymerization was continuously distilled out. After 1 h, the yield of depolymerization was 107%.

### Application example 102

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/100. The mixture was stirred at 160 °C and a pressure of 10 Pa reduced by an oil pump, and a product of cyclodepolymerization was continuously distilled out. After 1 h, the yield of depolymerization was 105%.

### Application example 103

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/100. The mixture was stirred at 160 °C and a pressure of 1000 Pa reduced by an oil pump, and a product of cyclodepolymerization was continuously distilled out. After 1 h, the yield of depolymerization was 98%.

### Application example 104

The Zn²⁺ [⁻OOC-poly(PDO)₁₀-OH]₂ prepared in step (2) of example 71 was used to catalyze the cyclodepolymerization of PPDO. The molar ratio of the terminal hydroxyl of the catalyst to the ester bond of PPDO was 1/100. The mixture was stirred at 160 °C and a pressure of 1*10⁴ Pa reduced by a water pump, and a product of cyclodepolymerization was continuously distilled out. After 1 h, the yield of depolymerization was 93%.

### Application example 105

The Zn²⁺ [⁻OOC-poly(L-LA)₁₀-OH]₂ prepared in step (2) of example 67 was used to catalyze the polycondensation and cyclodepolymerization of L-lactic acid. The molar ratio of the terminal hydroxyl of the catalyst to L-lactic acid was 1/50. The mixture was reacted at 80, 100, 120, and 140 °C successively at a pressure of 3000 Pa reduced by a water pump for 2 h, respectively, and the generated water was continuously distilled out. Then, the mixture was reacted at 140 °C and a pressure of 100 Pa reduced by an oil pump for 2 h, and the generated water was continuously distilled out to give an oligomer. Then, the resulting product was heated to 160 °C, and subjected to cyclodepolymerization at 1 Pa. The product was continuously generated and distilled out. After 3 h, the yield was 99%. The content of L-LA in the crude product was about 92%, and the content of meso-LA was about 1%.

### Application example 106

The Zn²⁺ [⁻OOC-poly(L-LA)₁₀-OH]₂ prepared in step (2) of example 67 was used to catalyze the polycondensation and cyclodepolymerization of L-methyl lactate. The molar ratio of the terminal hydroxyl of the catalyst to L-methyl lactate was 1/100. The mixture was reacted at 140 °C and atmospheric pressure for 4 h, and the generated methanol was continuously distilled out. Then, the pressure was slowly reduced to 3000 Pa, and the mixture was reacted for another 2 h, and the generated methanol was continuously distilled out to give an oligomer. Then, the resulting product was heated to 160 °C, and subjected to cyclodepolymerization at 100 Pa. The product was continuously generated and distilled out. After 3 h, the yield was 96%. The content of L-LA in the crude product was about 93%, and the content of meso-LA was about 1%.

### Application example 107

The Zn²⁺ [⁻OOC-poly(L-LA)₁₀-OH]₂ prepared in step (2) of example 67 was used to catalyze the polycondensation and cyclodepolymerization of L-ethyl lactate. The molar ratio of the terminal hydroxyl of the catalyst to L-ethyl lactate was 1/200. The mixture was reacted at 140 °C and atmospheric pressure for 6 h, and the generated ethanol was continuously distilled out. Then, the pressure was slowly reduced to 3000 Pa, and the mixture was reacted for another 2 h, and the generated ethanol was continuously distilled out to give an oligomer. Then, the resulting product was heated to 160 °C, and subjected to cyclodepolymerization at 10 Pa. The product was continuously generated and distilled out. After 3 h, the yield was 95%. The content of L-LA in the crude product was about 93%, and the content of meso-LA was about 1%.

### Application example 108

The Zn²⁺ [⁻OOC-poly(ε-CL)_{14.5}-OH]₂ prepared in step (2) of example 69 was used to catalyze the polycondensation and cyclodepolymerization of 6-hydroxyhexanoic acid. The molar ratio of the terminal hydroxyl of the catalyst to 6-hydroxyhexanoic acid was 1/20. The mixture was reacted at 80, 100, 120, and 140 °C successively at a pressure of 3000 Pa reduced by a water pump for 2 h, respectively, and the generated water was continuously distilled out. Then, the mixture was reacted at 160 °C and a pressure of 100 Pa reduced by an oil pump for 2 h, and the generated water was continuously distilled out to give an oligomer. Then, the resulting product was heated to 200 °C, and subjected to cyclodepolymerization at 1 Pa. The product was continuously generated and distilled out. After 4 h, the yield was 101%. In the crude product, the content of ε-CL monomers was about 81%, the content of ε-CL cyclic dimers was about 16%, and the content of ε-CL cyclic trimers was about 2%.

### Application example 109

The Zn²⁺ [⁻OOC-poly(ε-CL)_{14.5}-OH]₂ prepared in step (2) of example 69 was used to catalyze the polycondensation and cyclodepolymerization of methyl 6-hydroxyhexanoate. The molar ratio of the terminal hydroxyl of the catalyst to methyl 6-hydroxyhexanoate was 1/10. The mixture was reacted at 160 °C and atmospheric pressure for 2 h, and the generated methanol was continuously distilled out. Then, the pressure was slowly reduced to 3000 Pa, and the mixture was reacted for another 2 h, and the generated methanol was continuously distilled out to give an oligomer. Then, the resulting product was heated to 200 °C, and subjected to cyclodepolymerization at 100 Pa. The product was continuously generated and distilled out. After 4 h, the yield was 96%. In the crude product, the content of ε-CL monomers was about 80%, the content of ε-CL cyclic dimers was about 17%, and the content of ε-CL cyclic trimers was about 2%.

### Application example 110

The Zn²⁺ [⁻OOC-poly(ε-CL)_{14.5}-OH]₂ prepared in step (2) of example 69 was used to catalyze the polycondensation and cyclodepolymerization of ethyl 6-hydroxyhexanoate. The molar ratio of the terminal hydroxyl of the catalyst to ethyl 6-hydroxyhexanoate was 1/20. The mixture was reacted at 160 °C and atmospheric pressure for 4 h, and the generated ethanol was continuously distilled out. Then, the pressure was slowly reduced to 3000 Pa, and the mixture was reacted for another 4 h, and the generated ethanol was continuously distilled out to give an oligomer. Then, the resulting product was heated to 200 °C, and subjected to cyclodepolymerization at 10 Pa. The product was continuously generated and distilled out. After 4 h, the yield was 95%. In the crude product, the content of ε-CL monomers was about 78%, the content of ε-CL cyclic dimers was about 18%, and the content of ε-CL cyclic trimers was about 2%.

### Application example 111

The Zn²⁺ [⁻OOC-poly(GA)₁₀-OH]₂ prepared in step (2) of example 68 was used to catalyze the polycondensation and cyclodepolymerization of glycolic acid. The molar ratio of the terminal hydroxyl of the catalyst to glycolic acid was 1/100. The mixture was reacted at 100, 120, and 140 °C successively at a pressure of 3000 Pa reduced by a water pump for 3 h, respectively, and the generated water was continuously distilled out. Then, the mixture was reacted at 160 °C and a pressure of 100 Pa reduced by an oil pump for 2 h, and the generated water was continuously distilled out to give an oligomer of glycolic acid. Then, the resulting product was heated to 220 °C, and subjected to cyclodepolymerization at 1000 Pa. The product was continuously generated and distilled out. After 1 h, the yield was 95%.

### Application example 112

The Zn²⁺ [⁻OOC-poly(GA)₁₀-OH]₂ prepared in step (2) of example 68 was used to catalyze the polycondensation and cyclodepolymerization of methyl glycolate. The molar ratio of the terminal hydroxyl of the catalyst to methyl glycolate was 1/1000. The mixture was reacted at 140 °C and atmospheric pressure for 2 h, and the generated methanol was continuously distilled out. Then, the pressure was slowly reduced to 3000 Pa, and the mixture was slowly heated to 180 °C and reacted for 6 h. The generated methanol was continuously distilled out to give an oligomer of glycolic acid. The resulting product was cooled to room temperature, and then crushed and passed through a 40-mesh sieve. Then, the resulting product was extracted with xylene at 80 °C and atmospheric pressure (1*10⁵ Pa), and monomers were continuously generated and transferred to the extract (xylene). After 12 h, the depolymerization conversion rate was 31%.

### Application example 113

The Zn²⁺ [⁻OOC-poly(GA)₁₀-OH]₂ prepared in step (2) of example 68 was used to catalyze the polycondensation and cyclodepolymerization of ethyl glycolate. The molar ratio of the terminal hydroxyl of the catalyst to ethyl glycolate was 1/10000. The mixture was reacted at 140 °C and atmospheric pressure for 2 h, and the generated ethanol was continuously distilled out. Then, the pressure was slowly reduced to 3000 Pa, and the mixture was slowly heated to 180 °C and reacted for 6 h. The generated ethanol was continuously distilled out. Then, the mixture was heated to 220 °C and reacted for 0.5 h. The generated ethanol was continuously distilled out to give an oligomer of glycolic acid. Then, the resulting product was heated to 400 °C and subjected to cyclodepolymerization at 1*10⁴ Pa. The product was continuously generated and distilled out. After 1 h, the yield was 91%.

### Application example 114

The Zn²⁺ [⁻OOC-poly(δ-VL)₁₀-OH]₂ prepared in step (2) of example 70 was used to catalyze the polycondensation and cyclodepolymerization of 5-hydroxypentanoic acid. The molar ratio of the terminal hydroxyl of the catalyst to 5-hydroxypentanoic acid was 1/50. The mixture was reacted at 80, 100, 120, 140 and 160 °C successively at a pressure of 3000 Pa reduced by a water pump for 2 h, respectively, and the generated water was continuously distilled out. Then, the mixture was reacted at 160 °C and a pressure of 100 Pa reduced by an oil pump for 2 h, and the generated water was continuously distilled out to give an oligomer of 5-hydroxypentanoic acid. Then, the resulting product was subjected to cyclodepolymerization at 160 °C and 1 Pa. The product was continuously generated and distilled out. After 2 h, the yield was 105%.

### Application example 115

The Zn²⁺ [⁻OOC-poly(δ-VL)₁₀-OH]₂ prepared in step (2) of example 70 was used to catalyze the polycondensation and cyclodepolymerization of methyl 5-hydroxypentanoate. The molar ratio of the terminal hydroxyl of the catalyst to methyl 5-hydroxypentanoate was 1/100. The mixture was reacted at 160 °C and atmospheric pressure for 6 h, and the generated methanol was continuously distilled out. Then, the pressure was slowly reduced to 3000 Pa, and the mixture was reacted for another 2 h. The generated methanol was continuously distilled out to give an oligomer of 5-hydroxypentanoic acid. Then, the resulting product was subjected to cyclodepolymerization at 160 °C and 100 Pa. The product was continuously generated and distilled out. After 6 h, the yield was 100%.

### Application example 116

The Zn²⁺ [⁻OOC-poly(δ-VL)₁₀-OH] prepared in step (2) of example 70 was used to catalyze the polycondensation and cyclodepolymerization of ethyl 5-hydroxypentanoate. The molar ratio of the terminal hydroxyl of the catalyst to ethyl 5-hydroxypentanoate was 1/200. The mixture was reacted at 160 °C and atmospheric pressure for 6 h, and the generated ethanol was continuously distilled out. Then, the pressure was slowly reduced to 3000 Pa, and the mixture was reacted for another 2 h. The generated ethanol was continuously distilled out to give an oligomer of 5-hydroxypentanoic acid. Then, the resulting product was subjected to cyclodepolymerization at 160 °C and 10 Pa. The product was continuously generated and distilled out. After 6 h, the yield was 92%.

### Application example 117

The Al³⁺ (⁻OOC-C₁₇-OH)₃ prepared in step (2) of example 47 was used to catalyze the polycondensation and cyclodepolymerization of 12-hydroxyoctadecanoic acid. The molar ratio of the terminal hydroxyl of the catalyst to 12-hydroxyoctadecanoic acid was 1/100. The mixture was reacted at 80, 100, 120, 140 and 160 °C successively at a pressure of 3000 Pa reduced by a water pump for 2 h, respectively, and the generated water was continuously distilled out. Then, the mixture was reacted at 160 °C and a pressure of 100 Pa reduced by an oil pump for 2 h, and the generated water was continuously distilled out to give an oligomer of 12-hydroxyoctadecanoic acid. Then, the resulting product was heated to 220 °C, and subjected to cyclodepolymerization at 1 Pa. The product was continuously generated and distilled out. After 1.5 h, the yield was 95%.

### Application example 118

The Al³⁺ (⁻OOC-C₁₇-OH)₃ prepared in step (2) of example 47 was used to catalyze the polycondensation and cyclodepolymerization of methyl 12-hydroxyoctadecanoate. The molar ratio of the terminal hydroxyl of the catalyst to methyl 12-hydroxyoctadecanoate was 1/100. The mixture was reacted at 160 °C and atmospheric pressure for 6 h, and the generated methanol was continuously distilled out. Then, the pressure was slowly reduced to 3000 Pa, and the mixture was reacted for another 2 h. The generated methanol was continuously distilled out to give an oligomer of 12-hydroxyoctadecanoic acid. Then, the resulting product was heated to 220 °C, and subjected to cyclodepolymerization at 1 Pa. The product was continuously generated and distilled out. After 1.5 h, the yield was 91%.

### Application example 119

The Al³⁺ (⁻OOC-C₁₇-OH)₃ prepared in step (2) of example 47 was used to catalyze the polycondensation and cyclodepolymerization of ethyl 12-hydroxyoctadecanoate. The molar ratio of the terminal hydroxyl of the catalyst to ethyl 12-hydroxyoctadecanoate was 1/100. The mixture was reacted at 160 °C and atmospheric pressure for 6 h, and the generated ethanol was continuously distilled out. Then, the pressure was slowly reduced to 3000 Pa, and the mixture was reacted for another 2 h. The generated ethanol was continuously distilled out to give an oligomer of 12-hydroxyoctadecanoic acid. Then, the resulting product was heated to 220 °C, and subjected to cyclodepolymerization at 1 Pa. The product was continuously generated and distilled out. After 1.5 h, the yield was 89%.

### Application example 120

The Zn²⁺ [⁻OOC-poly(L-LA)₁₀-OH]₂ prepared in step (2) of example 67 was used to catalyze the cyclodepolymerization of a blend of PCL and PLLA (the mass ratio was 50/50). The molar ratio of the terminal hydroxyl of the catalyst to the ester bond of the polyester was 1/100. The mixture was stirred at 200 °C and a pressure of 100 Pa reduced by an oil pump, and a product of cyclodepolymerization was continuously distilled out. After 1 h, the yield of depolymerization was 51% (relative to the blend). In the product of depolymerization, the content of L-LA was 96%, and the content of ε-CL monomers and the cyclic oligomers thereof was 3%.

### Application example 121

The Zn²⁺ [⁻OOC-poly(L-LA)₁₀-OH]₂ prepared in step (2) of example 67 was used to catalyze the cyclodepolymerization of a block copolymer of L-LA and ε-CL (the molar ratio of the copolymer structural units was 57/43). The molar ratio of the terminal hydroxyl of the catalyst to the ester bond of the polyester was 1/100. The mixture was stirred at 200 °C and a pressure of 100 Pa reduced by an oil pump, and a product of cyclodepolymerization was continuously distilled out. After 1 h, the yield of depolymerization was 66% (relative to the copolymer). In the product of depolymerization, the content of L-LA was 95%, and the content of ε-CL monomers and the cyclic oligomers thereof was 7%.

### Application example 122

The Zn²⁺ [⁻OOC-poly(L-LA)₁₀-OH]₂ prepared in step (2) of example 67 was used to catalyze the cyclodepolymerization of a random copolymer of L-LA and PDO (the molar ratio of the copolymer structural units was 45/55). The molar ratio of the terminal hydroxyl of the catalyst to the ester bond of the polyester was 1/100. The mixture was stirred at 200 °C and a pressure of 100 Pa reduced by an oil pump, and a product of cyclodepolymerization was continuously distilled out. After 1 h, the yield of depolymerization was 93% (relative to the copolymer). In the product of depolymerization, the content of L-LA was 95%, and the content of PDO was 91%.

### Application example 123

The Zn²⁺ [⁻OOC-poly(L-LA)₁₀-OH]₂ prepared in step (2) of example 67 was used to catalyze the cyclodepolymerization of a blend of polybutylene adipate terephthalate (PBAT) and PLLA (the mass ratio was 30/70). The molar ratio of the terminal hydroxyl of the catalyst to the ester bond of the polyester was 1/100. The mixture was stirred at 200 °C and a pressure of 100 Pa reduced by an oil pump, and a product of cyclodepolymerization was continuously distilled out. After 1 h, the yield of depolymerization was 101% (relative to PLLA). In the product of depolymerization, the content of L-LA was 99%.

### Application example 124

The Zn²⁺ [⁻OOC-poly(L-LA)₁₀-OH]₂ prepared in step (2) of example 67 was used to catalyze the cyclodepolymerization of a complex of calcium carbonate and PLLA (the mass ratio was 38/62). The molar ratio of the terminal hydroxyl of the catalyst to the ester bond of the polyester was 1/100. The mixture was stirred at 200 °C and a pressure of 100 Pa reduced by an oil pump, and a product of cyclodepolymerization was continuously distilled out. After 1 h, the yield of depolymerization was 105% (relative to PLLA). In the product of depolymerization, the content of L-LA was 99%.

### Application example 125

The Zn²⁺ [⁻OOC-poly(L-LA)₁₀-OH]₂ prepared in step (2) of example 67 was used to catalyze the cyclodepolymerization of a complex of starch and PLLA (the mass ratio was 20/80). The molar ratio of the terminal hydroxyl of the catalyst to the ester bond of the polyester was 1/100. The mixture was stirred at 200 °C and a pressure of 100 Pa reduced by an oil pump, and a product of cyclodepolymerization was continuously distilled out. After 1 h, the yield of depolymerization was 98% (relative to PLLA). In the product of depolymerization, the content of L-LA was 99%.

### Application example 126

The Zn²⁺ [⁻OOC-poly(PDO)₁₀-OH]₂ prepared in step (2) of example 71 was used to catalyze the cyclodepolymerization of PPDO polyurethane. The molar ratio of the terminal hydroxyl of the catalyst to the ester bond of PPDO was 1/100. The mixture was stirred at 180 °C and a pressure of 100 Pa reduced by an oil pump, and a product of cyclodepolymerization was continuously distilled out. After 2 h, the yield of depolymerization was 95% (relative to PPDO).

### Application example 127

The Zn²⁺ [⁻OOC-poly(PDO)₁₀-OH]₂ prepared in step (2) of example 71 was used to catalyze the cyclodepolymerization of a composite material of PPDO and carbon fiber. The molar ratio of the terminal hydroxyl of the catalyst to the ester bond of PPDO was 1/100. The mixture was stirred at 180 °C and a pressure of 100 Pa reduced by an oil pump, and a product of cyclodepolymerization was continuously distilled out. After 2 h, the yield of depolymerization was 93% (relative to PPDO).

### Application example 128

The Zn²⁺ [⁻OOC-poly(PDO)₁₀-OH]₂ prepared in step (2) of example 71 was used to catalyze the cyclodepolymerization of chemically cross-linked PPDO. The molar ratio of the terminal hydroxyl of the catalyst to the ester bond of PPDO was 1/100. The mixture was stirred at 180 °C and a pressure of 100 Pa reduced by an oil pump, and a product of cyclodepolymerization was continuously distilled out. After 2 h, the yield of depolymerization was 82% (relative to the mass of PPDO).

## Claims

1. A catalyst for polyester depolymerization or cyclic ester synthesis, which is a long-chain catalyst containing both a terminal ion group and a terminal hydroxyl structure, wherein the terminal ion group is either a cation connected to the long-chain structure or an anion connected to the long-chain structure, and the structural formulas are respectively as follows: 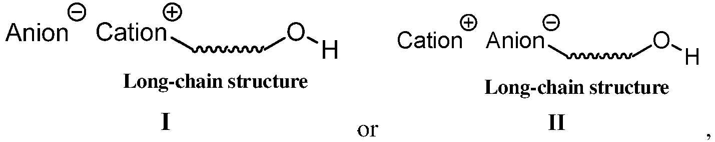 the number of carbon atoms in the main chain of the long-chain structure is 8 or more; the long-chain structure of the structural formula I is any one of a long alkyl chain, an aliphatic polyester chain and a polyether-ester chain; the long-chain structure of the structural formula II is any one of a long alkyl chain, an aliphatic polyester chain, a polyether-ester chain and a polyether chain.

2. The catalyst for polyester depolymerization or cyclic ester synthesis according to claim 1, wherein the long alkyl chain of the long-chain structure in the catalyst is an n-octane chain, an n-pentadecane chain or an α-hexyldodecane chain; the aliphatic polyester chain is poly(ethylene succinate), polybutylene succinate, poly(hexylene succinate), poly(ethylene adipate), polybutylene adipate, poly(hexylene adipate), polylactic acid, polyglycolide, polycaprolactone or polyvalerolactone; the polyether-ester chain is polydioxanone, poly(3,4-dihydro-2H-benzo[1,4]dioxepine-2-one) or poly(4-phenyl-3,4-dihydro-2H-benzo[1,4]dioxepine-2-one); and the polyether chain is polyethylene glycol or polytetrahydrofuran.

3. The catalyst for polyester depolymerization or cyclic ester synthesis according to claim 1 or 2, wherein the cation in the structural formula I of the catalyst is an imidazolium ion or a thiazolium ion, and the anion is a halide ion or a halogenated metal chloride; the anion in the structural formula II is an organic carboxylate ion or an organic sulfonate ion, and the cation is a metal ion or a quaternary ammonium ion.

4. The catalyst for polyester depolymerization or cyclic ester synthesis according to claim 3, wherein the imidazolium ion in the structural formula I of the catalyst is a 1-long-chain substituted-3-methylimidazolium ion or a 1-long-chain substituted-3-butylimidazolium ion, the thiazolium ion is a 3-long-chain substituted-4-methylthiazolium ion, a 3-long-chain substituted benzothiazolium ion or a 3-long-chain substituted thiazolium ion; the halide ion in the structural formula I is a chloride ion or a bromide ion; the halogenated metal chloride in the structural formula I is chlorinated stannous chloride, chlorinated aluminum chloride, chlorinated zinc chloride, brominated ferric chloride or chlorinated ferric chloride; the organic carboxylate ion in the structural formula II is an aliphatic carboxylate ion; the organic sulfonate ion in the structural formula II is an aliphatic sulfonate ion; the metal ion in the structural formula II is a sodium ion, a potassium ion, a magnesium ion, a calcium ion, a zinc ion, a tin ion, an iron ion or an aluminum ion; the quaternary ammonium ion in the structural formula II is a tetramethylammonium ion or a tetraethylammonium ion.

5. A method of preparing the catalyst for polyester depolymerization or cyclic ester synthesis according to claim 1, wherein the process steps and conditions for preparing the catalyst of structural formula I are as follows:
(1) subjecting a hydroxyl-containing alkyl halide to a quaternization reaction with 0.8-1.2 times the molar amount of a compound containing an imidazole or thiazole structure to give a hydroxyl-containing imidazolium halide, a hydroxyl-containing thiazolium halide, a hydroxyl-containing imidazolium halide catalyst with a long alkyl chain in the structural formula I, or a hydroxyl-containing thiazolium halide catalyst with a long alkyl chain in the structural formula I;
(2) subjecting the hydroxyl-containing imidazolium halide, hydroxyl-containing thiazolium halide, hydroxyl-containing imidazolium halide catalyst with a long alkyl chain in the structural formula I or hydroxyl-containing thiazolium halide catalyst with a long alkyl chain in the structural formula I obtained in step (1) to a Lewis acid-base neutralization reaction with 1-3 times the molar amount of a Lewis acidic metal ion chloride, to convert the anion of the above salt from a halide ion to halogenated metal chloride, thereby giving another type of hydroxyl-containing imidazolium salt, another type of hydroxyl-containing thiazolium salt, another type of hydroxyl-containing imidazolium salt catalyst with a long alkyl chain in the structural formula I or another type of hydroxyl-containing thiazolium salt catalyst with a long alkyl chain in the structural formula I;
(3) subjecting the other type of hydroxyl-containing imidazolium salt, the other type of hydroxyl-containing thiazolium salt, the other type of hydroxyl-containing imidazolium salt catalyst with a long alkyl chain in the structural formula I or the other type of hydroxyl-containing thiazolium salt catalyst with a long alkyl chain in the structural formula I obtained in step (2) to a ring-opening polymerization reaction with 1-100 times the molar amount of a cyclic ester or a cyclic ether-ester to give an imidazolium salt catalyst with a long-chain structure of polyester or polyether-ester in the structural formula I or a thiazolium salt catalyst with a long-chain structure of polyester or polyether-ester in the structural formula I; or mixing the other type of hydroxyl-containing imidazolium salt, the other type of hydroxyl-containing thiazolium salt, the other type of hydroxyl-containing imidazolium salt catalyst with a long alkyl chain in the structural formula I or the other type of hydroxyl-containing thiazolium salt catalyst with a long alkyl chain in the structural formula I obtained in step (2) with 1-100 times the molar amount of a hydroxy acid or with 1-100 times the molar amount of a dibasic acid and 1-100 times the molar amount of a diol to give an imidazolium salt or thiazolium salt catalyst with a long-chain structure of aliphatic polyester in the structural formula I through an esterification polycondensation reaction,
the process steps and conditions for preparing the catalyst of structural formula II are as follows: (1) mixing a hydroxyl-containing organic carboxylic acid or a hydroxyl-containing organic sulfonic acid with an alkali metal hydroxide, an alkaline earth metal hydroxide or a quaternary ammonium base at a molar ratio of carboxylate or sulfonate to hydroxide of 0.8-1.2, and preparing a hydroxyl-containing organic carboxylate, organic sulfonate, a hydroxyl-containing organic carboxylate catalyst with a long alkyl chain in the structural formula II or a hydroxyl-containing organic sulfonate catalyst with a long alkyl chain in the structural formula II whose cation is an alkali metal ion, an alkaline earth metal ion or a quaternary ammonium ion through a Bronsted-Lowry acid-base neutralization reaction; or mixing a cyclic ester with an alkali metal hydroxide, an alkaline earth metal hydroxide or a quaternary ammonium base at a molar ratio of cyclic ester to hydroxide of 0.8-1.2, and preparing a hydroxyl-containing organic carboxylate or a hydroxyl-containing organic sulfonate catalyst with a long alkyl chain in the structural formula II whose cation is an alkali metal ion, an alkaline earth metal ion or a quaternary ammonium ion through an ester bond alkaline hydrolysis reaction;
(1) mixing the organic carboxylate, organic sulfonate, hydroxyl-containing organic carboxylate catalyst with a long alkyl chain in the structural formula II or hydroxyl-containing organic sulfonate catalyst with a long alkyl chain in the structural formula II whose cation is an alkali metal ion obtained in step (1) with a Lewis acidic metal ion chloride at a molar ratio of carboxylate or sulfonate to chloride ion of 0.8-2.2, and preparing another type of hydroxyl-containing organic carboxylate, organic sulfonate, hydroxyl-containing organic carboxylate catalyst with a long alkyl chain in the structural formula II or hydroxyl-containing organic sulfonate catalyst with a long alkyl chain in the structural formula II whose cation is a Lewis acidic metal ion through a metathesis reaction;
(3) mixing the hydroxyl-containing organic carboxylate, organic sulfonate, hydroxyl-containing organic carboxylate catalyst with a long alkyl chain in the structural formula II, or hydroxyl-containing organic sulfonate catalyst with a long alkyl chain in the structural formula II whose cation is an alkali metal ion, an alkaline earth metal ion, a quaternary ammonium ion or a Lewis acidic metal ion obtained in step (2) with 1-100 times the molar amount of a cyclic ester or a cyclic ether-ester to give a catalyst with a long-chain structure of polyester or polyether-ester in the structural formula II through a ring-opening polymerization reaction, or with 1-100 times the molar amount of a hydroxy acid or 1-100 times the molar amount of a dibasic acid and 1-100 times the molar amount of a diol to give a catalyst with a long-chain structure of polyester in the structural formula II through an esterification polycondensation reaction,
or, the process steps and conditions for preparing the catalyst of structural formula II are as follows:
(1) mixing an aliphatic polyester or polyether-ester with an alkali metal hydroxide, an alkaline earth metal hydroxide or a quaternary ammonium base at a molar ratio of ester bond to hydroxide of 1-100, and then performing an ester bond alkaline hydrolysis reaction at a temperature higher than the melting point of the corresponding polymer to give an aliphatic polyester or polyether-ester catalyst containing a terminal carboxylate and a terminal hydroxyl whose cation is an alkali metal ion, an alkaline earth metal ion or a quaternary ammonium ion in the structural structure II; or mixing a polyether containing a terminal carboxylic acid and a terminal hydroxyl with an alkali metal hydroxide, an alkaline earth metal hydroxide or a quaternary ammonium base at a molar ratio of terminal carboxylate to hydroxide of 0.8-1.2, and then performing a Bronsted-Lowry acid-base neutralization reaction at a temperature higher than the melting point of the corresponding polymer to give an aliphatic polyester, polyether-ester or polyether catalyst containing a terminal carboxylate and a terminal hydroxyl whose cation is an alkali metal ion, an alkaline earth metal ion or a quaternary ammonium ion in the structural formula II;
(2) mixing the aliphatic polyester, polyether-ester or polyether catalyst containing a terminal carboxylate and a terminal hydroxyl whose cation is an alkali metal ion in the structural formula II obtained in step (1) with a Lewis acidic metal ion chloride at a molar ratio of carboxylate to chloride ion of 1-2, and then performing a metathesis reaction at a temperature higher than the melting point of the corresponding polymer to give an aliphatic polyester, polyether-ester or polyether catalyst containing a terminal carboxylate and a terminal hydroxyl whose cation is a Lewis acidic metal ion in the structural formula II.

6. The method of preparing the catalyst for polyester depolymerization or cyclic ester synthesis according to claim 5, wherein, in the process steps of preparing the catalyst of structural formula I, the hydroxyl-containing alkyl halide used in the quaternization reaction is 8-chloro-1-octanol, 3-chloro-1-propanol or 2-bromo-1-ethanol, and the imidazole or thiazole structure-containing compound used is 1-methylimidazole, 1-butylimidazole, 4-methylthiazole, thiazole or benzothiazole, the reaction temperature is 60-120 °C, and the reaction time is 12-48 h;
in the process steps of preparing the catalyst of the structural formula I, the MClₙ used in the Lewis acid-base neutralization reaction is zinc chloride, stannous chloride, aluminum chloride or ferric chloride, the reaction temperature is 25-120 °C, and the reaction time is 0.5-12 h;
in the process steps of preparing the catalyst of the structural formula I or the process steps of preparing the catalyst of the structural formula II, the cyclic ester or cyclic ether-ester used in the ring-opening polymerization reaction is lactide, glycolide, ε-caprolactone, δ-valerolactone, p-dioxanone, 3,4-dihydro-2H-benzo[1,4]dioxepine-2-one or 4-phenyl-3,4-dihydro-2H-benzo[1,4]dioxepine-2-one, the reaction temperature is 60-220 °C, and the reaction time is 1-48 h;
in the process steps of preparing the catalyst of the structural formula I or the process steps of preparing the catalyst of the structural formula II, the dibasic acid used in the esterification polycondensation reaction is 1,4-butanedioic acid or 1,6-hexanedioic acid, the diol used is 1,2-ethanediol, 1,4-butanediol or 1,6-hexanediol, the reaction temperature is 80-220 °C, the reaction time is 8-24 h, and the reaction pressure is 1-1*10⁵ Pa;
in the process steps of preparing the catalyst of the structural formula II, the hydroxyl-containing organic carboxylic acid used in the Bronsted-Lowry acid-base neutralization reaction is 12-hydroxyoctadecanoic acid, 6-hydroxyhexanoic acid, 18-hydroxyoctadecanoic acid or 8-hydroxyoctanoic acid, the hydroxyl-containing organic sulfonic acid used is 4-hydroxy-1-butanesulfonic acid, 3-hydroxy-1-propanesulfonic acid or 2-hydroxyethanesulfonic acid, the alkali metal hydroxide used is sodium hydroxide or potassium hydroxide, the alkaline earth metal hydroxide used is magnesium hydroxide or calcium hydroxide, the quaternary ammonium base used is tetramethylammonium hydroxide or tetraethylammonium hydroxide, the reaction temperature is 25-180 °C, and the reaction time is 0.5-12 h;
in the process steps of preparing the catalyst of the structural formula II, the cyclic ester used in the ester bond alkaline hydrolysis reaction is cyclopentadecanolide or ε-CL, the alkali metal hydroxide used is sodium hydroxide or potassium hydroxide, the alkaline earth metal hydroxide used is magnesium hydroxide or calcium hydroxide, the quaternary ammonium base used is tetramethylammonium hydroxide or tetraethylammonium hydroxide, the reaction temperature is 25-180 °C, and the reaction time is 0.5-12 h;
in the process steps of preparing the catalyst of the structural formula II, the MClₙ used in the metathesis reaction is ZnCl₂, SnCl₂, AlCl₃ or FeCl₃, the reaction temperature is 25-220 °C, and the reaction time is 0.5-12 h;
in the process steps of preparing the catalyst of the structural formula II, the aliphatic polyester used in the ester bond alkaline hydrolysis reaction is PES, PBS, PHS, PEA, PBA, PHA, PLA, PGA, PCL or PVL, the polyether-ester used is PPDO, PBDXO or PDBXOP, the reaction temperature is 60-220 °C, and the reaction time is 1-48 h;
in the process steps of preparing the catalyst of the structural formula II, the polyether used in the Bronsted-Lowry acid-base neutralization reaction is PEG or PTHF, the reaction temperature is 25-120 °C, and the reaction time is 0.5-12 h.

7. A method of recovering monomers or monomers and oligomers, comprising catalyzing the hydrolysis, alcoholysis or cyclodepolymerization of a polyester in the presence of the catalyst according to claim 1.

8. The method according to claim 7, comprising: mixing the polyester, water or alcohol, and catalyst according to a proportion, heating the mixture to 40-200 °C at 0.1-2 MPa for a hydrolysis or alcoholysis reaction for 1-12 h, and then recovering a solution of the corresponding monomers or monomers and oligomers in water or alcohol; or heating the mixture to 80-400 °C at 1-1*10⁴ Pa for cyclodepolymerization for 0.5-12 h, and simultaneously distilling or extracting the mixture to separate the generated cyclic ester monomers or cyclic ester monomers and cyclic oligomers from the reaction system; wherein the ratio of polyester to water or alcohol is 100:50-2000 wt%, and the molar ratio of the catalyst to the ester bond in the polyester is 0.01-10 mol% based on the terminal hydroxyl.

9. A method of synthesizing a cyclic ester monomer or a cyclic ester monomer and a cyclic oligomer, comprising subjecting a hydroxy acid or a hydroxy ester to a polycondensation and cyclization reaction in the presence of the catalyst according to claim 1.

10. The method according to claim 9, comprising:
(1) first, mixing the catalyst with a hydroxy acid or hydroxy ester at a molar ratio of 0.01-10 mol% based on the terminal hydroxyl, and then heating the mixture to 80-220 °C at 100-1*10⁵ Pa for a polycondensation reaction for 4-12 h, and simultaneously distilling the mixture to remove the water or alcohol generated in the reaction system to give an oligomer of hydroxy acid;
(2) heating the oligomer of hydroxy acid obtained in step (1) to 80-400 °C at 1-1*10⁵ Pa for a cyclodepolymerization reaction for 0.5-4 h, and simultaneously distilling or extracting the resulting mixture to separate the generated cyclic ester monomers or cyclic ester monomers and cyclic oligomers from the reaction system.

11. The method according to claim 10, wherein the hydroxy acid is glycolic acid, lactic acid, 6-hydroxyhexanoic acid, 5-hydroxypentanoic acid or 12-hydroxyoctadecanoic acid, and the hydroxy ester is any one of methyl glycolate, ethyl glycolate, methyl lactate, ethyl lactate, methyl 6-hydroxyhexanoate, ethyl 6-hydroxyhexanoate, methyl 5-hydroxypentanoate, ethyl 5-hydroxypentanoate, methyl 12-hydroxyoctadecanoate and ethyl 12-hydroxyoctadecanoate.
